# EUROPÄISCHE PATENTSCHRIFT

(11) **EP 1 919 535 B1**
(45) Veröffentlichungstag und Bekanntmachung des Hinweises auf die Patenterteilung: **12.10.2016**
(21) Anmeldenummer: 06763031.9
(22) Anmeldetag: 18.08.2006
(51) Int. Cl.: A61M 1/10, A61M 1/16, B01D 63/02, F04D 29/04, F04D 13/06

(54) **VORRICHTUNG ZUR AN- UND/ODER ABREICHERUNG VON STOFFEN IN EINER FLÜSSIGKEIT**
DEVICE FOR ENRICHING AND/OR DEPLETING MATERIALS IN A LIQUID
DISPOSITIF POUR ENRICHIR ET/OU APPAUVRIR UN FLUIDE EN SUBSTANCES

(30) Priorität: 18.08.2005 DE 102005039446
(43) Veröffentlichungstag der Anmeldung: 14.05.2008
(73) Patentinhaber: Xenios AG, 74076 Heilbronn (DE)
(72) Erfinder: STRAUSS, Andreas, 52064 Aachen (DE); AKDIS, Mustafa, 35799 Merenberg (DE)
(74) Vertreter: Castell, Klaus
(86) Internationale Anmeldenummer: PCT/EP2006/008178
(87) Internationale Veröffentlichungsnummer: WO 2007/020106

(56) Entgegenhaltungen:
- EP-A2- 0 611 580
- WO-A2-97/16213
- DE-A1- 2 251 176
- DE-A1- 10 341 221
- JP-A- 2 041 172
- US-A- 5 308 314

## Beschreibung

Die Erfindung betrifft eine Vorrichtung zum Anreichern und/oder Abreichern von Stoffen in Blut.

### Stand der Technik

In der DE 42 38 884 wird ein System dargestellt, das aus den einzelnen Komponenten Oxygenator, Wärmetauscher, Blutfilter und Blutreservoir aufgebaut ist, die durch Schläuche zum Transport des Blutes miteinander verbunden werden.

Ebenso sind Membranoxygenatoren mit integrierten Membranelementen und einem integrierten Wärmetauscher, wobei das Wärmetauscherelement fest im Oxygenator fixiert ist, im Stand der Technik zu finden, wie in der EP0507722 dargestellt.

Eine weitere Ausführungsform eines Oxygenators ist in der DE69317763 beschrieben. Dort wird ein modular aufgebauter, integrierter Einweg-Blutoxygenator beschrieben, der über ein austauschbares Wärmetauscherelement verfügt.

Eine Vorrichtung zur Behandlung von Flüssigkeiten, insbesondere Blut, bekannt aus der EP 0765683 und der US005817279 besteht beispielsweise aus mehreren Kammern, die aus Rohren gebildet werden. Eine kompakte Bauweise wird unter anderem dadurch erzielt, dass ein Zyklon teilweise in einem Rohr angeordnet ist.

Aus dem Stand der Technik sind weiterhin zahlreiche Kreiselpumpen zur Förderung von Blut bekannt.

So zeigt die Patentschrift DE 101 08 810 A1 eine Blutpumpe, bei der das Schaufelrad über elektronisch geregelte Magnetlager berührungslos abgestützt wird. Zur berührungslosen Rotorlagerung wird neben der Antriebsenergie zusätzliche Energie benötigt.

Die Patentschrift US 5,840,070 zeigt eine Pumpe, bei der die Abstützung des Rotors über zahlreiche Magnete erfolgt, die sowohl im Schaufelrad als auch im Pumpengehäuse untergebracht sind.

Weiterhin zeigt die Patentschrift US 6,116,862 eine Blutpumpe mit einem Schaufelrad, welches zur Rotorstabilisierung zwei verschleißbehaftete mechanische Gleitlager verwendet. Eines dieser Rotorlager beinhaltet eine Kugel-Kalottenlagerung auf der Rückseite des Schaufelrades zur axialen Rotorstabilisierung. Mit Hilfe des ersten Lagers werden insbesondere die Anziehungskräfte aus der magnetischen Kupplungsvorrichtung aufgefangen. Das zweite Rotorlager beinhaltet eine Welle-Buchse-Lagerung zur radialen Stabilisierung des Rotors sowie zur Aufnahme von Kippkräften, die aus der Magnetkupplung resultieren.

Der Erfindung liegt die Aufgabe zugrunde, ein mobiles, kompaktes, extrakorporales Oxygenationssystem bereitzustellen, das ein möglichst geringes Füllvolumen aufweist und dessen Fremdkontaktoberfläche minimiert ist. Außerdem soll dieses System eine gute Handhabbarkeit aufweisen, schnell einsatzbereit, blut schonend und wieder verwendbar sein.

DE 103 41 221 offenbart den nächstliegenden Stand der Technik.

Erfindungsgemäß wird diese Aufgabe durch eine Vorrichtung mit den Merkmalen des unabhängigen Anspruches 1 gelöst. Vorteilhafte Weiterbildungen der Vorrichtung ergeben sich aus den Unteransprüchen 2-38.

Weitere Vorteile, Besonderheiten und zweckmäßige Weiterbildungen der Erfindung ergeben sich aus den Unteransprüchen und der nachfolgenden Darstellung bevorzugter Ausführungsbeispiele anhand der Abbildungen.

Die Erfindung sieht vor, eine Vorrichtung zur An- und Abreicherung von Stoffen in einer Flüssigkeit mit einem Membranmodul auszustatten, welches im Wesentlichen aus konzentrischen Körpern besteht und ein Separationselement zur An- und Abreicherung einer Flüssigkeit aufweist, wobei in dem Separationselement der ab- und anzureichernde Stoff und außerhalb des Separationselements die Flüssigkeit geführt ist.

Zweckmäßigerweise beinhaltet eine besonders bevorzugte Ausführungsform der Erfindung ein kompaktes, mobiles Oxygenationssystem mit geringem Füllvolumen, minimierter Membranoberfläche sowie integrierter, strömungsmechanisch gelagerter, auswechselbarer und gegebenenfalls wieder verwendbarer Blutpumpe sowie optimierter Gaszufuhr zur intrakorporalen oder extrakorporalen Oxygenierung von Patienten.

Die Auswechselbarkeit ist auch dann möglich, während die Vorrichtung mit mindestens einem Gefäß eines Patienten verbunden ist. Dies bedeutet, dass bei dem Auswechseln der Antriebseinheit der Betrieb der Vorrichtung nur sehr kurzfristig, beispielsweise zwischen einer Sekunde und 30 Sekunden, vorzugsweise höchstens 20 Sekunden unterbrochen wird. Dies bedeutet bezüglich der physiologischen Wirkung eine Auswechselbarkeit während des Betriebes.

Die Vorrichtung enthält ein Antriebsmodul, welches eine Antriebseinheit zum Antrieb eines die Flüssigkeit fördernden Förderelements aufweist.

In einer besonders bevorzugten Ausführungsform der Vorrichtung ist das Förderelement ein Rotor.

Die Vorrichtung zeichnet sich dadurch aus, dass das Antriebsmodul bei flüssigkeitsdichtem Verschluss in das Membranmodul einschiebbar und rausnehmbar ist.

Hierdurch ist das Antriebsmodul in das Membranmoduls in dieses während des Betriebs einschiebbar und rausnehmbar.

Es ist zweckmäßig, das Fördermodul zur Förderung der Flüssigkeit in axialer Verlängerung des Antriebsmoduls anzuordnen.

Dies hat den Vorteil, dass die Trennbarkeit zwischen dem Antriebsmodul und dem Fördermodul verbessert wird.

Ferner ist es zweckmäßig, das Förderelement und ein es umgebendes Gehäuse, vorzugsweise ein Rotorgehäuse, voneinander trennbar anzuordnen.

In diesem Fall verbleibt das Förderelement - vorzugsweise ein Rotor - bei einem Auswechseln des Antriebsmoduls in der Flüssigkeit.

Es ist ferner möglich, bei einem flüssigkeitsdichten Abschluss des Förderelementes - vorzugsweise einem Rotor - gegenüber Zuleitungen der Flüssigkeit und/oder Ableitungen der Flüssigkeit eine Auswechselbarkeit des Förderelementes zu ermöglichen.

Durch die Anordnung der Antriebseinheit innerhalb der Flüssigkeit wird eine Erwärmung der Flüssigkeit ermöglicht.

Diese Flüssigkeit befindet sich vorzugsweise nicht in einem direkten Kontakt mit der Antriebseinheit, sondern ist von diesem durch eine zusätzliche Trennwand getrennt.

Bei den Körpern kann es sich um beliebige, jedoch konzentrisch anordenbare geometrische Gebilde handeln. Zur Verringerung des Platzbedarfes ist eine Gestaltung in radialsymmetrischen Formen, insbesondere als Kugeln, Ellipsoiden oder Zylindern bevorzugt.

Es ist zweckmäßig, das Separationselement so auszustatten, dass es hohle Fasern aus semipermeablem Material zur Anreicherung und/oder Abreicherung der Flüssigkeit aufweist.

Der Einsatz von hohlen Fasern aus semipermeablem Material ist vorteilhaft. Diese Fasern ermöglichen besonders wirksame Trennprozesse durch eine Diffusion.

Eine bevorzugte Ausführungsform der Erfindung sieht einen Einsatz wenigstens einer semipermeablen Membran vor. Der Begriff "semipermeabel" bezeichnet hierbei eine Ausgestaltung der Membran, bei der ein Durchtritt einer ersten Substanz, vorzugsweise von Sauerstoff und/ oder CO₂, ermöglicht wird, während ein Durchtritt einer anderen Substanz - vorzugsweise Wasser - verhindert wird.

Ferner ist es vorteilhaft, dass das Separationselement zur An- und Abreicherung einer Flüssigkeit mit einem semipermeablen Material ausgestattet ist.

Vorzugsweise enthält das semipermeable Material Fasermembranen, wobei der abzureichernde Stoff zwischen den Fasern und der anzureichernde Stoff innerhalb der Hohlfasern angeordnet ist.

Hierdurch wird die zur Trennwirkung verfügbare Oberfläche erhöht.

Durch eine Schräganordnung von Fasern werden Turbulenzen der Flüssigkeit, insbesondere des Blutes, erzeugt. Hierdurch wird der Stoffaustausch und damit die Trennwirkung erhöht.

Zur Verbesserung der Auswechselbarkeit ist es zweckmäßig, dass das Antriebsmodul an wenigstens einer Stirnseite einen Schnellverschluss aufweist.

Vorzugsweise befindet sich der Schnellverschluss am Bodenelement des Oxygenationssystems.

Der Schnellverschluss kann, um eine sichere Verriegelung zu gewährleisten und unbeabsichtigtes Öffnen zu verhindern, in einer besonderen Ausführungsform ein Bajonettverschluss sein.

Weitere Beispiele geeigneter Schnellverschlüsse sind Schraubverschlüsse und Klemmverschlüsse.

Auch Magnetverschlüsse sind zweckmäßig.

Der Verschluss ist vorzugsweise an einer Stirnseite des Körpers angeordnet. Hierdurch wird eine sichere Fixierung des Antriebselementes während des Betriebes der Vorrichtung ermöglicht.

Vorzugsweise handelt es sich bei dem Antriebselement um einen Motor oder eine Turbine. Der Begriff Motor umfasst alle Antriebe, die geeignet sind, ein Drehmoment auf das Förderelement zu übertragen. Der Einsatz eines Elektromotors ist besonders bevorzugt. Die Turbine ist vorzugsweise pneumatisch angetrieben.

Zweckmäßigerweise ist zwischen der Antriebseinheit und einer Rotoreinheit ein Dämpfungselement angeordnet.

Das Dämpfungselement ist zweckmäßigerweise so ausgestaltet, dass es Stöße beim Einbringen des Antriebselementes verringert.

Vorzugsweise wird das Dämpfungselement durch einen luftgefüllten Hohlraum gebildet, der mit einer oder mehreren kleinen Ausweichöffnungen für Luft verbunden ist.

Hierbei sind die Austrittsöffnungen für die Luft so bemessen, dass das Einbringen des Antriebselements um wenigstens 0,5 Sekunden, vorzugsweise zwischen 1 Sekunde und 10 Sekunden verzögert wird.

Bei besonders bevorzugten Abmessungen zu verdrängender Luftvolumina, insbesondere zwischen ca. 10 ml und ca. 500 ml, vorzugsweise maximal 200 ml, beträgt der einzustellende Luftaustrittsstrom zur Erzielung der gewünschten Verzögerung zwischen 1 ml/s und 500 ml/s. Bei besonders vorteilhaften Luftvolumina zwischen 10 ml und 200 ml und einer gewünschten Mindestverzögerung zwischen 2 Sekunden und 10 Sekunden beträgt der einzustellende Luftaustrittsstrom 5 ml/s bis 100 ml/s.

Gegenüber derzeit bekannten ECMO-Systemen weist die Erfindung mehrere Vorteile auf.

Besonders bevorzugte Ausführungsformen der erfindungsgemäßen Vorrichtung zeichnen sich wie folgt aus: Es ist möglich, auf Grund der guten Handhabbarkeit und des geringen Gewichtes das System nicht nur stationär, sondern auch mobil in Rettungsfahrzeugen, Notarztwagen oder Hubschraubern direkt zum Unfallort zu transportieren. Das Gerät kann leicht von einer Person getragen und bedient werden und ist durch seine Kompaktheit in Ärztekoffern unterzubringen.

Die bevorzugte Aufteilung in ein wieder verwertbares Blutpumpenantriebsmodul - die Antriebseinheit - und eine Einmal-Einheit - ein die Antriebseinheit umgebendes Modul, vorzugsweise ein Membranmodul - ermöglicht vielfältige Einsätze der erfindungsgemäßen Vorrichtung.

Da die Blutpumpe direkt in dem Oxygenator integriert ist, trägt sie gleichzeitig zur Temperierung des Blutes bei.

Dies macht ein externes Wärmetauscheraggregat überflüssig. Es ist jedoch gleichfalls möglich, zur weiteren Erwärmung des Blutes ein derartiges Wärmetauscheraggregat einzusetzen, wobei jedoch Ausführungsformen ohne das weitere Wärmetauscheraggregat wegen ihrer Kompaktheit noch vorteilhafter sind. Es sind keine Schlauchverbindungen und Konnektoren nötig, um die Komponenten miteinander zu verbinden. Das erhöht die Sicherheit und reduziert zusätzlich Fremdkontaktoberfläche und Füllvolumen (das ist dasjenige Volumen, mit dem das System mit Fremdblut oder Blutersatzflüssigkeit aufgefüllt werden muss, um die Luft aus dem System zu verdrängen und damit Embolien zu vermeiden). Der Antrieb der integrierten Blutpumpe ist jederzeit - auch während des Betriebes - schnell und leicht zu entnehmen und, da er nicht mit Blut in Berührung kommt, wieder verwertbar. Durch das geringe Füllvolumen ist die Erfindung gerade auch für die Anwendung bei Kindern besonders gut geeignet.

Ferner ist es zweckmäßig, wenigstens einen Sensor einzusetzen. Besonders zweckmäßige Beispiele sind Sensoren für die Messung der Temperatur des Blutes, der Durchflussgeschwindigkeit des Blutes und/oder der Blutgase.

Darüber hinaus ist es zweckmäßig, wenigstens einen Drucksensor einzusetzen. Der Einsatz eines Drucksensors ermöglicht es, einen Abgleich zwischen einem tatsächlichen Druck und vorgebbaren Solldrücken zu ermöglichen.

Es ist besonders vorteilhaft, wenigstens einen Sensor im Bereich eines Körpers oder innerhalb eines der Körper einzusetzen. Ferner ist eine Anordnung wenigstens eines Sensors im Bereich eines Deckels vorteilhaft.

Das Membranmodul der Erfindung weist mindestens zwei Körper auf.

Bei den Körpern handelt es sich beispielhaft um konzentrisch zueinander platzierte Zylinder, wobei:
- zwischen den Zylindern hohle, mikroporöse Membranen platziert sind und die Räume zwischen den Zylindern an den Stirnseiten flüssigkeitsdicht verschlossen sind.
- der innerste der drei Zylinder an der dem Fördermodul gegenüberliegenden Seite einen Deckel mit einem Schnellverschluss aufweist.

Eine zweckmäßige Ausführungsform des Fördermoduls ist, um Füllvolumen zu reduzieren unter Berücksichtigung von Sicherheitsaspekten, geometrisch so ausgestaltet, dass es im Innenraum des innersten der mindestens zwei Körper platzierbar und in diesen während des Betriebs einschiebbar und herausnehmbar ist.

Eine besonders bevorzugte Ausführungsform des Fördermoduls ist so ausgestaltet, dass sein radialer Außendurchmesser kleiner als der Radius des Innenraums des innersten Zylinders ist.

Eine zweckmäßige Ausführungsform des Fördermoduls zeichnet sich dadurch aus, dass es bei der Montage der Vorrichtung in den Innenraum des innersten Zylinders einschiebbar ist.

Es ist vorteilhaft, um den Anschluss einer Doppellumenkanüle zu ermöglichen, auf eine der Stirnseiten eines der Zylinder der Erfindung einen Aufsatz aufzusetzen, welcher eine Zuführung und eine Abführung zur Zu- und Abführung der Flüssigkeit aufweist, die koaxial zueinander angeordnet sind.

Um den Gastransport - insbesondere den CO₂-Abtransport zu optimieren -, ist es zweckmäßig, mikroporöse Membranen, die zwischen dem ersten und zweiten Körper und/oder zwischen dem zweiten und einem dritten Körper platziert sind, mit jeweils einer Stoffzu- und einer Stoffableitung auszustatten.

Derartige Ausführungsformen sind insbesondere dann vorteilhaft, wenn es sich um rotationssymmetrische Körper, insbesondere Kugeln, Ellipsoide oder Zylinder handelt.

Aus diesem Grunde sind in einer bevorzugten Ausführung die Gaszuführungen der zwischen dem ersten und zweiten Körper platzierten Membranen und/oder zwischen dem zweiten und dritten Körper platzierten Membranen an gegenüberliegenden angeordneten Stirnseiten der Zylinder angeordnet.

Um Bluttraumatisierung und Thrombogenese zu minimieren sowie die Modularität zu gewährleisten, ist es zweckmäßig, die Vorrichtung so auszustatten, dass die Anreicherung und/oder Abreicherung von Stoffen in einer Flüssigkeit, mit einer Antriebseinheit zum Antrieb eines die Flüssigkeit fördernden Förderelements so ausgestaltet ist, dass sie die Kraft und/oder das Drehmoment von der Antriebseinheit auf das Förderelement berührungslos überträgt.

Dies geschieht in einer besonders bevorzugten Ausführungsform dadurch, dass die Vorrichtung so ausgestattet ist, dass die Anreicherung und/oder Abreicherung von Stoffen in einer Flüssigkeit, mit einer Antriebseinheit zum Antrieb eines die Flüssigkeit fördernden Förderelements so ausgestaltet ist, dass sie die Kraft und/oder das Drehmoment von der Antriebseinheit auf das Förderelement mittels einer Magnetkupplung überträgt.

Eine zweckmäßige Ausführung der Vorrichtung sieht vor, in einer im Wesentlichen zylinderförmigen Aufnahme eine Antriebseinheit aufzunehmen, die im Betrieb Wärme abgibt.

Eine zweckmäßige Ausführung der Vorrichtung sieht vor, in einer im Wesentlichen zylinderförmigen Aufnahme eine Antriebseinheit aufzunehmen, die ein Elektromotor ist.

Eine zweckmäßige Ausführung der Vorrichtung sieht vor, in einer im Wesentlichen zylinderförmigen Aufnahme eine Antriebseinheit aufzunehmen, die eine Turbine ist.

Eine zweckmäßige Ausführung der Vorrichtung sieht vor, in einer im Wesentlichen zylinderförmigen Aufnahme eine Antriebseinheit aufzunehmen, die in wärme leitendem Kontakt mit einer zylinderförmigen Aufnahme steht, an deren Außenseite Flüssigkeit entlang geführt wird.

Es ist vorteilhaft, dass die Vorrichtung ein Förderelement aufweist, das in radialer Richtung mit einer strömungsmechanischen Lagerung und/oder einer magnetischen Lagerung ausgestattet ist, die in einen Raum zwischen Förderelement und umgebendem Gehäuse durch einen dem Hauptförderstrom entgegensetzten Sekundärstrom bewirkt wird.

Ein Vorteil einer derartigen Lagerung liegt im einfachen Aufbau und Design der Fluidlagerung. Die Fluidlagerung ist in einer besonders bevorzugten Ausführungsform so ausgestattet, dass, das Förderelement bei vorliegenden Lagerspaltbreiten (ca. 100 *µ*m bis 1000 *µ*m) im Vergleich zu konventionellen hydrodynamischen Lagern (ca. 10 *µ*m bis 100 *µ*m) auch bei größeren Lagerspaltbreiten betrieben werden kann. Dadurch ist insbesondere der Vorteil gegeben, dass die Schädigung der Blutzellen bei der vorliegenden Erfindung wesentlich geringer ist als bei konventionellen hydrodynamischen Lagern, so dass das in der vorliegenden Erfindung beschriebene Oxygenationssystem wesentlich patientenfreundlicher betrieben werden kann.

Es ist vorteilhaft, dass die Vorrichtung ein Förderelement aufweist, das in der dem Antriebsmodul zugewandten Seite in einem körperhaften Axiallager aufgelagert ist.

Die Vorrichtung beinhaltet einen Oxygenator. Es ist ferner möglich, dass die Vorrichtung selbst ein Oxygenator ist.

Es ist vorteilhaft, die Vorrichtung so auszugestalten, dass sie in ihrem Innenraum eine Blutpumpe umschließt, welche in einer radialen Richtung strömungsmechanisch stabilisiert ist.

Eine besonders bevorzugte Ausführungsform der Vorrichtung zeichnet sich dadurch aus, dass sie eine axiale, radiale oder diagonale Kreiselblutpumpe aufweist.

Die erfindungsgemäße Oxygenator ist so ausgestattet dass er einen außen liegenden Faserbündel und einen innen liegenden Faserbündel aufweist, wobei zwischen dem außen liegenden und dem innen liegenden Faserbündel eine elektromagnetischer Antriebseinheit integriert ist, welche eine radiale Magnetkupplung für ein zentrales innen liegenden Laufrad beinhaltet.

Der Oxygenator weißt einen außen liegenden Faserbündel und einen innen liegenden Faserbündel auf, wobei zwischen dem außen liegenden und dem innen liegenden Faserbündel eine elektromagnetischer Antriebseinheit integriert ist, welche eine radiale Magnetkupplung für den zentral innen liegenden Laufrad hervorbringt.

Eine besonders bevorzugte Ausführungsform der Vorrichtung zeichnet sich dadurch aus, dass in der Magnetkupplung wirkende Magnetkräfte eine Stabilisierung des Laufrades im Pumpengehäuse bewirken.

Ferner ist es zweckmäßig, einen erfindungsgemäßen Oxygenator so auszustatten, dass er einen außen liegenden Faserbündel und einen innen liegenden Faserbündel aufweist, wobei zwischen dem außen liegenden und dem innen liegenden Faserbündel eine elektromagnetischer Antriebseinheit integriert ist, welche sowohl das außen liegende als auch den innen liegende Faserbündel aufwärmt.

Es ist vorteilhaft, eine erfindungsgemäße Vorrichtung mit wenigstens zwei Antriebsmodulen auszustatten.

Eine Anordnung von zwei Antriebsmodulen in Reihenschaltung ermöglicht einen Einsatz kleinerer und kompakterer Antriebsmodule.

Es ist besonders vorteilhaft, zwei Antriebsmodule in Parallelschaltung anzuordnen. In diesem Fall kann während eines Ausfalls eines der Antriebsmodule ein anderes Antriebsmodul weiter betrieben werden. Ferner ist es möglich, ein Antriebsmodul auszutauschen, während das andere Antriebsmodul gewünschte Antriebsleistungen erbringt.

Weitere Vorteile, Besonderheiten und bevorzugte Weiterbildungen der Erfindung sind Gegenstand der Unteransprüche und der nachfolgenden Darstellung bevorzugter Ausführungsbeispiele anhand der Zeichnungen.

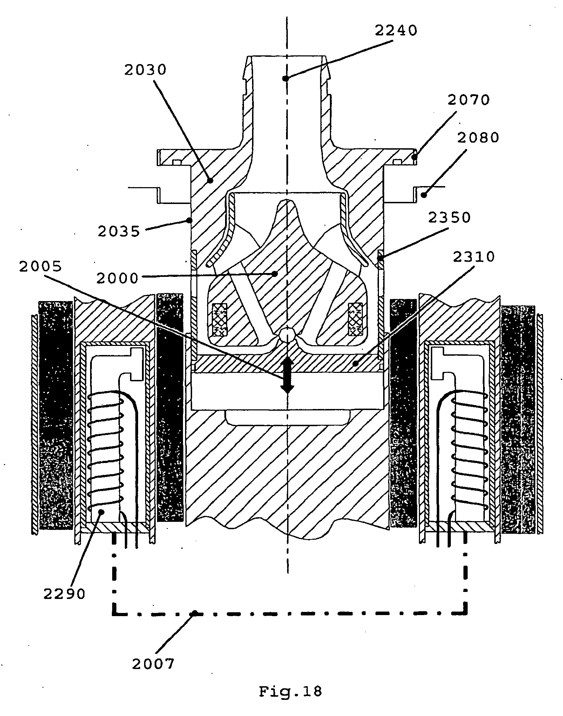

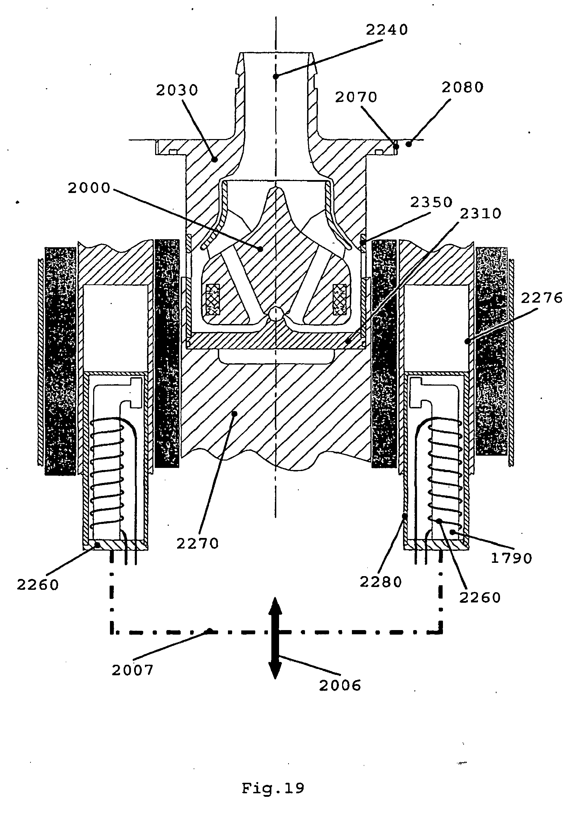

Figuren 17 bis 19 zeigen ein Ausführungsbeispiel der Erfindung. Die Figuren 1 bis 16 zeigen verschiedene Aspekte der Vorrichtung, wobei die Erfindung in Anspruch 1 definiert ist.
- Figur 1:: einen Schnitt durch ein erfindungsgemäßes Oxygenationssystem mit Darstellung der Strömungslagerung der Blutpumpe und der Schnellverschlussvorrichtung.
- Figur 2:: eine Detailansicht des Antriebmoduls mit Schnellverschluss im Längsschnitt.
- Figur 3:: eine Ansicht des Deckels mit Aussparung und Nuten.
- Figur 4:: eine Detailansicht der Strömungslagerung im Schnitt.
- Figur 5:: einen Schnitt durch das Oxygenationssystem mit eingezeichnetem Blutstromverlauf und Strömungsverlauf des im doppelten Gegenstrom geführten Gasstroms.
- Figur 6:: einen Schnitt durch den Anschluss des Oxygenationssystems für die Konnektierung an den Patienten mittels Doppellumenkanüle.
- Figur 7:: eine dreidimensionale Ansicht durch ein weiteres erfindungsgemäßes Oxygenationssystem mit Darstellung der Strömungslagerung der Blutpumpe und der Schnellverschlussvorrichtung.
- Figur 8:: eine Schnittdarstellung des Fördermoduls.
- Figur 9:: einen Schnitt durch das in Fig. 7 dargestellte Oxygenationssystem mit Darstellung der Module.
- Figur 10:: eine schematische Darstellung der Funktionsweise sowie der Blut- und Gasströmungen.
- Figur 11: Pumpeneinheit des Oxygenators einschließlich der Strömungs- und Gasführung; Antrieb über einen integrierten Elektromotor mit permanentmagnetischer Axialkupplung.
- Figur 12: eine Ausführung des Oxygenators gemäß Fig. 11 mit axial beweglicher Pumpeneinheit.
- Figur 13: eine Ausführung des Oxygenators gemäß Fig. 11 mit axial beweglicher Antriebseinheit.
- Figur 14: Pumpeneinheit des Oxygenators einschließlich der Strömungs- und Gasführung; Antrieb über einen integrierten Elektromotor mit elektromagnetischer Radialkupplung.
- Figur 15: eine Ausführung des Oxygenators gemäß Fig. 14 mit axial beweglicher Pumpeneinheit
- Figur 16: eine Ausführung des Oxygenators gemäß Fig. 14 mit axial beweglicher Antriebseinheit
- Figur 17: die Erfindung: eine Pumpeneinheit des Oxygenators einschließlich Strömungs- und Gasführung; Antrieb über einen integrierten Elektromotor mit elektromagnetischer Radialkupplung; Statoreinheit des Antriebes ist beidseitig mit Oxygenatorfasern umgeben.
- Figur 18: eine Ausführung des Oxygenators gemäß Fig. 17 mit axial beweglicher Pumpeneinheit.
- Figur 19: eine Ausführung des Oxygenators gemäß Fig. 17 mit axial beweglicher Antriebseinheit.

Im Folgenden wird die Erfindung anhand von Ausführungsbeispielen näher erläutert.

Zweckmäßigerweise weist die Vorrichtung Mittel auf, die die Einleitung und Abführung von Fluiden in das System ermöglichen. Bei den Mitteln handelt es beispielsweise um einen Einlasskanal und einen Auslasskanal. Es können jedoch auch mehrere Einlasskanäle und/oder Auslasskanäle vorgesehen sein.

Das Blut fließt durch einen Bluteinlass in den Oxygenator und wird zunächst durch eine integrierte Blutpumpe geleitet. Anschließend strömt das Blut in eine Kammer und von da über einen Blutauslass aus dem System.

Blutein- und Auslass des Oxygenationssystems können koaxial ausgebildet werden, so dass eine doppellumige Kanüle direkt - ohne Adapter - angeschlossen werden kann. Dies hat den Vorteil, Überschneidungen im Schlauchsystem zu vermeiden. Hierdurch werden Strömungsabrisse, Totwassergebiete oder Thrombenbildung verhindert. Ferner verkürzt sich die Zeit bis zur Einsatzbereitschaft des Oxygenationssystems.

Dies ist gerade in kritischen Situationen ein entscheidender Vorteil.

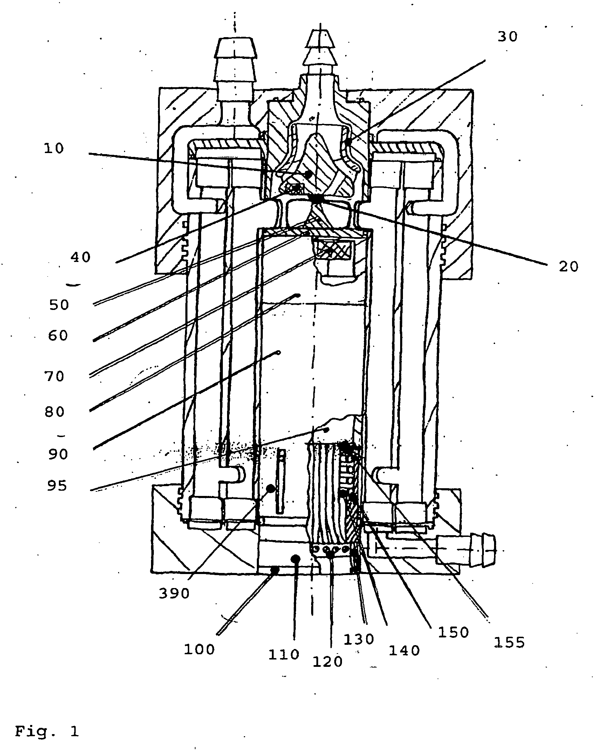

Die Figur 1 zeigt einen Schnitt durch das im Wesentlichen um seine Längsachse rotationssymmetrische Oxygenationssystem mit integrierter Blutpumpe. Das System besteht im Wesentlichen aus einem Oxygenator und einer in diesem platzierten Blutpumpe. Der Oxygenator besteht aus einem Membranmodul, das aus den Zylindern 250, 260 und 270 sowie den darin befindlichen Fasern 330 und 340 aufgebaut ist, sowie den stirnseitigen Deckelelementen 230 und 240. Die Blutpumpe ist aus Förder- 10 - 60 und Antriebsmodul 70-150 zusammengesetzt. Das Fördermodul wird zur Montage in den innersten Zylinder 250 des Oxygenators geschoben. Das Fördermodul wird durch die Schulterausformungen an der Öffnung des Deckels 230 und die Mutter 170 fixiert. Das Antriebsmodul weist eine Schnellverschlussvorrichtung auf, die vorliegend als Bajonettverschluss, bestehend aus Druckknopf 100, einer Steckerhalterung 110, die den Stecker 120 fixiert, einem Ringelement 130, einer Feder 140 und den Rückhohlstiften 150, ausgebildet ist. Beim Einschub der Einheit in das Oxygenationssystem rastet der Verschluss in die dafür vorgesehenen Nuten 400 im Deckel 240 ein. Durch die Feder wird das Antriebsmodul gegen das Rotormodul gepresst und dadurch axial fixiert. Auf Knopfdruck und bei gleichzeitiger Drehung rastet der Bajonettverschluss wieder aus. Durch die Feder springt der Druckknopf 100 aus dem Oxygenatorgehäuse, so dass die Antriebseinheit dem Oxygenator auf einfache und schnelle Weise entnommen werden kann.

Das Schaufelrad 10 wird über den Motor 90 mit Hilfe einer Magnetkupplung 40, 70 angetrieben. Als axiale Lagerung des Schaufelrades 10 dient die Stützlagerung 20, welches die Anziehungskräfte aus der Magnetkupplung 40, 70 aufnimmt. Die radiale Stabilisierung des Schaufelrades 10 wird berührungslos über die strömungsmechanische Lagerung 30 bewirkt, welche die Kippkräfte aus der Magnetkupplung 40, 70 aufnimmt.

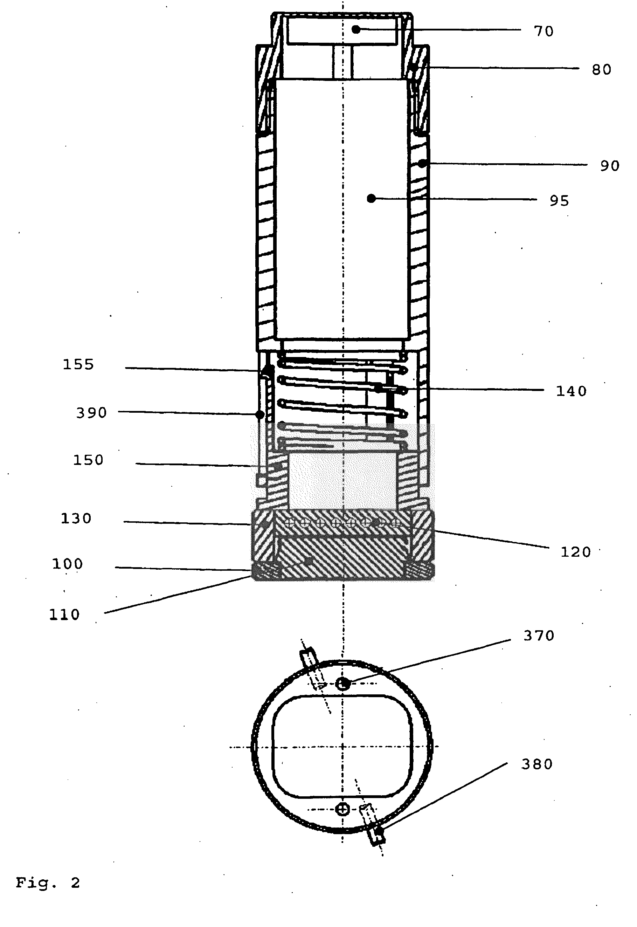

Figur 2 zeigt das Antriebsmodul, bestehend aus Motor 95 mit Magnetkupplung 70, achtpoligem Kabel und Stecker 120. Der Motor mit Magnetkupplung ist vollständig vom Motorgehäuse 90 mit aufgeschraubtem Motordeckel 80 umschlossen. Das Kabel mit Stecker wird aus dem Gehäuse herausgeführt. Dieser ist in einer mehrteiligen Vorrichtung 100-150 durch zwei Schrauben 370 so fixiert, dass eine direkte Konnektierung mit dem Gegenstecker der Stromversorgung von außen möglich ist. Das mittlere Bauteil der dreiteiligen Steckerhalterung ist ein Ring 130 mit zwei Bohrungen, in die zwei Passstifte 380 für die Renkverbindung eingesetzt sind. Das größte Bauteil der Steckerhalterung 150 ist auf der dem Stecker abgewandten Seite mit drei Haken 150 ausgestattet. Diese greifen in die Schlitze 390 im Motorgehäuse, halten so das Antriebsmodul zusammen und lassen trotzdem eine axiale Verschiebung gegenüber dem Motorgehäuse 90 zu. Die Feder 140 im Innern des Antriebsmoduls sorgt im eingebauten Zustand für eine spielfreie axiale Positionierung des Motorgehäuses 90 im Zylinder 250. Beim Lösen der Renkverbindung drückt sie den Stecker inklusive Halterung aus dem inneren Zylinder 250 heraus, während die restlichen Bauteile des Antriebsmoduls zunächst unverändert von der Anziehungskraft der Magnetkupplung in der Oxygenationseinheit gehalten werden. Die das System bedienende Person kann nun an der Steckerhalterung anfassen und das Antriebsmodul aus dem Oxygenator entfernen. Der Motor ist zweifach gegen Rotation in Folge des Motormoments gesichert. Unmittelbar wird das Moment vom Motorgehäuse aufgenommen, in das der Motor über den Motordeckel eingeklemmt ist. Das Motorgehäuse ist wiederum über die Renkverbindung gegen Verdrehen gesichert, das Drehmoment wird von dem Elementenpaar Nut/Stift aufgenommen.

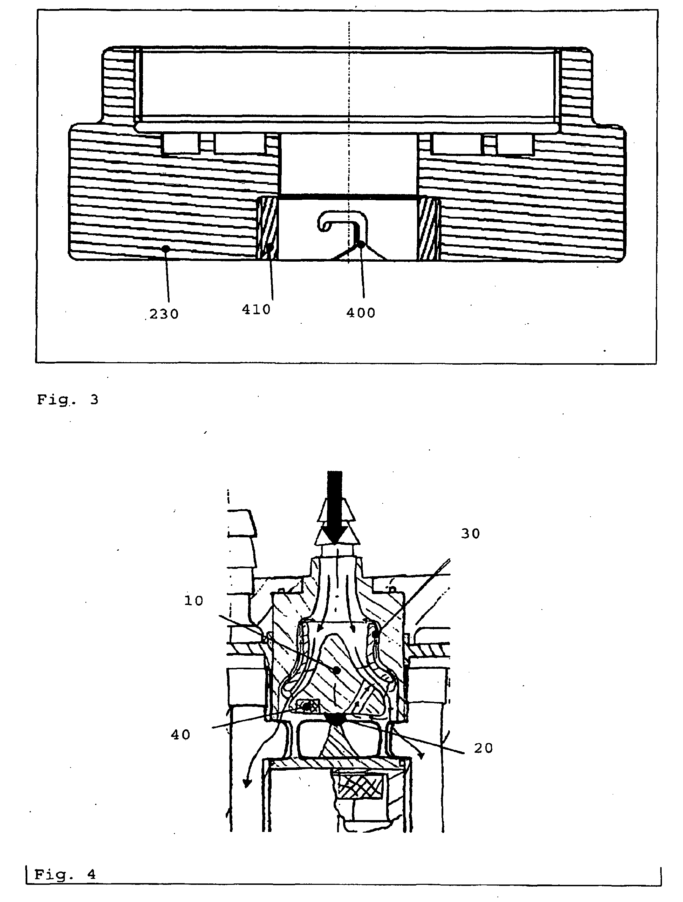

Figur 3 zeigt einen zweiteilig aufgebauten, steckerseitigen Deckel: Eine Renknut 400 ist in einem separaten Zylinder 410 untergebracht, was die Fertigung erheblich vereinfacht. Der Zylinder ist mit dem Deckel 230 über ein Gewinde verbunden.

Figur 4 zeigt die Strömungsführung im Schaufelradbereich sowie im strömungsmechanischen Lager 30. Die vom Schaufelrad 10 geförderte Strömung wird vorwiegend in den Strömungskanal zwischen dem ersten Zylinder 250 und dem zweiten 260 weitergeleitet. Ein Teil dieser Strömung wird nach Verlassen des Schaufelrades 10 in den Ringkanal zwischen dem Schaufelrad 10 und dem umgebenden Gehäuse abgezweigt. Diese retrograd gerichtete Strömung bewirkt eine radiale Stabilisierung des Schaufelrades 10.

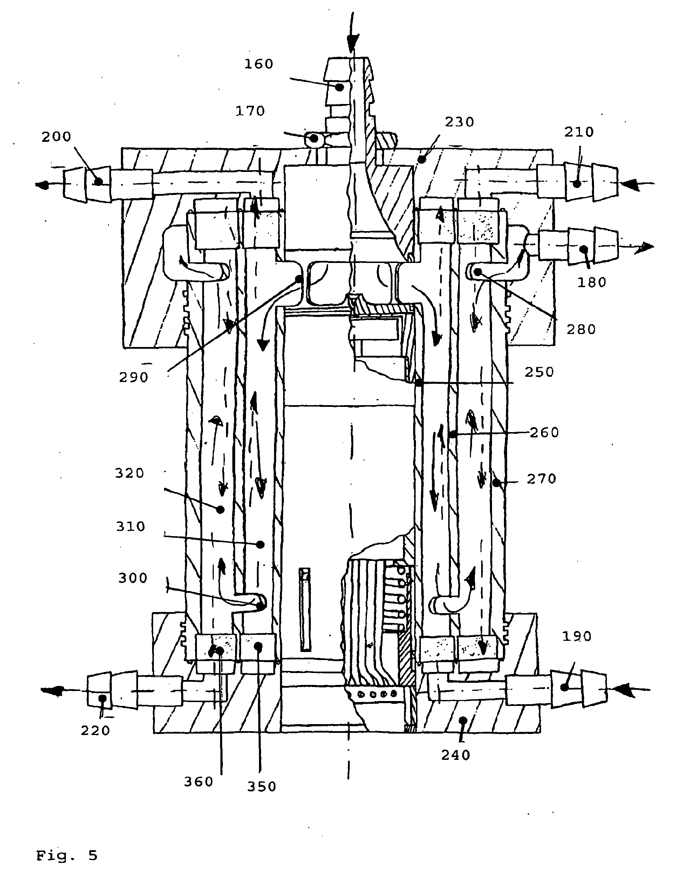

In Figur 5 ist dargestellt, wie Blutstrom (durchgezogene Linien) und Gasstrom (gestrichelte Linien) zweimal zueinander im Gegenstrom geführt werden. Das Blut strömt durch den Einlass 160 in den Oxygenator. Dort strömt es - wie durch die Pfeile angedeutet - zunächst durch die Öffnung 290 im ersten Zylinder 250 in die erste Kammer 310, die an den Enden durch die Verklebung 350 verschlossen ist, hinein und dort an den semipermeablen Hohlfasermembranen 330 vorbei. Anschließend strömt es, nachdem es die Aussparungen 300 im zweiten Zylinder 260 passiert hat, in der zweiten Kammer 320, die auch an den Enden verklebt ist, in die entgegengesetzte Richtung. Durch die Passagen 280 im dritten Zylinder 270 strömt das Blut in den Blutdeckel 230 und verlässt das System durch den Blutauslass 180. Die doppelte Gegenstromoxygenierung wird dadurch ermöglicht, dass Sauerstoff zunächst durch den Gaseinlass 190 in das System eintritt und anschließend durch diejenigen Membranen in Kammer 310 strömt, die zwischen erstem Zylinder 250 und zweitem Zylinder 260 angeordnet sind. Dieser Gasstrom verlässt das Oxygenierungssystem durch den Auslass 200. Ein zweiter Gasstrom 210 wird zeitgleich durch die Membranen in Kammer 320, zwischen zweitem Zylinder 260 und drittem Zylinder 270, geleitet und strömt durch den zweiten Gasauslass 220 wieder hinaus.

Die mehrmalige Zufuhr von frischem Sauerstoff gewährleistet einen effektiven Gasaustausch. Insbesondere begünstigt diese bevorzugte Ausführungsform der Erfindung den Austausch von Kohlendioxyd, dessen Abtransport besonders wichtig ist. In der abgebildeten Ausführungsform wird dem System Sauerstoff bzw. ein Luftgemisch an zwei Stellen im Gegenstrom zugeführt. Weitere Vorteile dieser Anordnung liegen in der flexibleren Dosierung, Steuerung und Regelung der Gaszufuhr. So kann je nach Krankheitsbild in der ersten Kammer zunächst reiner Sauerstoff zugeführt werden und dann in der zweiten Kammer ein definiertes Luftgemisch, um beispielsweise den CO₂-Abtransport zu dosieren. Denkbar ist auch die kombinierte Zufuhr von Sauerstoff in der ersten Kammer und eines Narkosegases in der zweiten. Ebenso kann zunächst beatmet werden und anschließend können in der zweiten Kammer toxische Substanzen - eine mögliche Ursache für Lungenversagen - entfernt werden. Dies geschieht beispielsweise mittels einer Dialyse des Blutstroms.

Zweckmäßigerweise weist die Vorrichtung Mittel auf, die die Einleitung und Abführung von Fluiden in das System ermöglichen. Beispielsweise sind dies Konnektoren oder Öffnungen. In einer besonderen Ausführungsform weist die Erfindung eine spezielle Strömungsführung für Sauerstoff und Kohlendioxyd auf, die den Stoffaustausch zwischen Gas- und Blutstrom im doppelten, direkten Gegenstromprinzip ermöglicht. Dazu wird sowohl im Inneren der semipermeablen Hohlfasermembranen 330 und 340 (stellvertretend ist jeweils eine Membran dargestellt), die sich in den Kammern 310 und 320 befinden und diese ausfüllen, Sauerstoff geleitet. Dies ist möglich, da die integrierte Blutpumpe, die mit einem Mittel zum besonders schnellen Ein- und Ausbau ausgestattet ist, mit dem Zylinder 250 in wärmeleitendem Kontakt steht und über diesen an das am Zylinder 250 vorbeifließende Blut Wärme zur Temperierung abgibt. Eine zusätzliche Temperierung ist möglich, jedoch bei besonders bevorzugten Ausführungsformen nicht erforderlich.

Somit kann das Fasermaterial der inneren Kammer 310 in einer besonderen Ausführungsform zur Oxygenierung und insbesondere zur Entfernung von Kohlendioxyd genutzt werden. Der Entfernung von Kohlendioxyd kommt gerade bei lungenkranken Patienten eine besondere Bedeutung zu. Die Elimination kann durch höhere Gasvolumenströme gesteigert werden. Die Höhe der Volumenströme ist durch die Druckverhältnisse im Oxygenator limitiert (Emboliegefahr). Durch die erfindungsgemäße Anordnung kann die Abatmung von CO₂ gegenüber herkömmlichen Oxygenatoren gesteigert werden.

Die integrierte Blutpumpe ist mit einem Mittel ausgestattet, das es ermöglicht, die Pumpe jederzeit - auch während des Betriebes - rasch auszuwechseln. In einer beispielhaften Ausführung kann dies ein Klemmverschluss- oder ein Schraubverschlussmechanismus sein. In einer besonders bevorzugten Ausführungsform weist die erfindungsgemäße Vorrichtung einen Schnellverschlussmechanismus auf.

Der Schnellverschlussmechanismus weist in einer besonders zweckmäßigen Ausführungsform Mittel zum Erzeugen einer Rückschnellkraft auf, beispielsweise ein elastisch verformbares Material oder eine Feder.

In einer besonders bevorzugten Ausführungsform weist der Schnellverschlussmechanismus ein Federelement auf. Das Federelement gewährleistet, dass das Antriebsaggregat so fixiert wird, dass der Motor mit Magnetkupplung an das Rotormodul gepresst werden kann und auch bei Entkoppeln der Kupplung das Antriebsaggregat seine Position beibehält.

Zur Verriegelung weist der Schnellverschluss Mittel auf, die ein schnelles und sicheres Einführen, sowie eine beschleunigte Entnahme ermöglichen. Beispielsweise beschleunigt das Federelement die Entnahme des Antriebselementes.

In einer besonderen Ausführungsform ist der Schnellverschluss ein Bajonettverschluss. Durch das Federelement springt bei Entriegelung des Antriebs der Bajonettverschluss heraus, so dass der Antrieb in Notsituationen auch während des Betriebes schnell entnommen werden kann. Dadurch wird die Sicherheit des Patienten gewährleistet.

Das Blut fließt durch einen Bluteinlass 160 in den Oxygenator und wird zunächst durch die integrierte Blutpumpe durch die Aussparung 290 im innersten Zylinder 250 in die Kammer 310 zwischen Zylinder 250 und Zylinder 260 geleitet. Anschließend strömt das Blut durch die Passagen 300 in Zylinder 260 in die Kammer 320, die aus Zylinder 260 und 270 gebildet wird, und von da durch die Öffnungen 280 in Zylinder 270 über den Blutauslass 180 aus dem System. Die Kammern sind nahezu vollständig mit semipermeablem Fasermaterial 330 und 340 gefüllt, so dass das Blut an den Membranen vorbeifließt und ein Stofftransport durch Diffusion stattfinden kann.
Durch die beschriebene, spezielle Anordnung wird das Konzentrationsgefälle erhöht und der Stofftransport bzw. Gasaustausch verbessert, was gerade für die miniaturisierte, kompakte Ausführung des Oxygenationssystems von großer Wichtigkeit ist.

Blutein- und Auslass des Oxygenationssystems können koaxial ausgebildet werden, so dass eine doppellumige Kanüle direkt - ohne Adapter - angeschlossen werden kann. Dies hat den Vorteil, Überschneidungen im Schlauchsystem zu vermeiden und die Zeit bis zur Einsatzbereitschaft des Oxygenationssystems zu verkürzen. Dies ist gerade in kritischen Situationen ein wichtiger Vorteil.

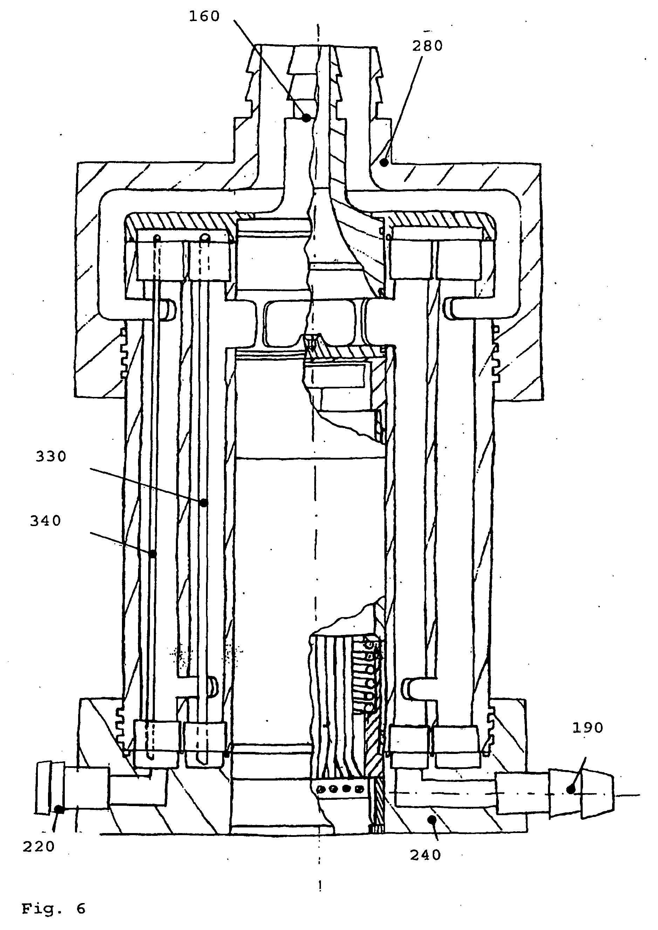

Figur 6 zeigt den koaxialen Anschluss für eine Doppellumenkanüle. Über dem Bluteinlass 160 wird ein speziell ausgeführter Blutdeckel 280 angeordnet. Dieser Deckel 280 kann optional verwendet werden.

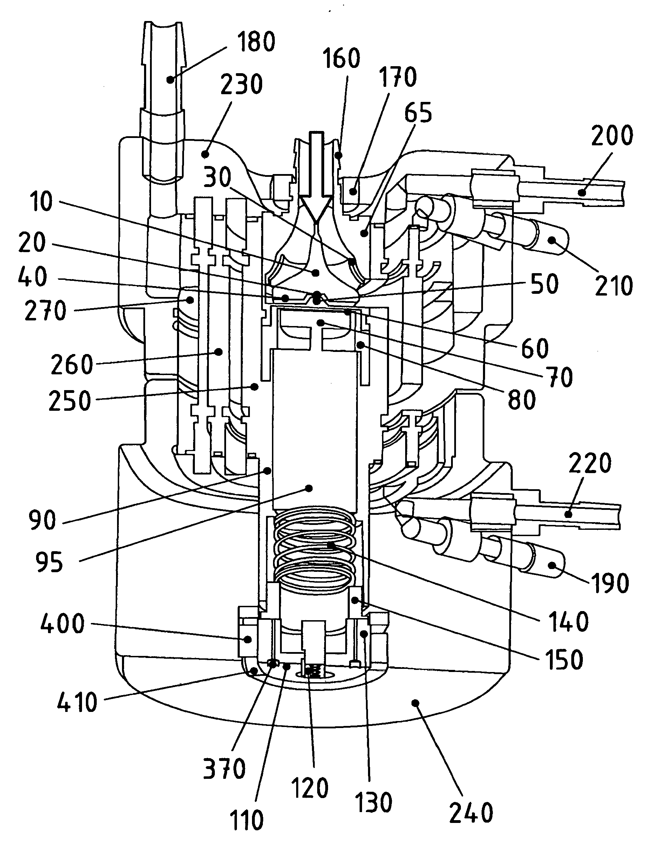

Figur 7 zeigt einen Schnitt durch eine weitere Ausführungsform des Oxygenationssystems mit integrierter Blutpumpe. Das System besteht im Wesentlichen aus einem Oxygenator und einer in diesem platzierten Blutpumpe. Der Oxygenator besteht aus einem Membranmodul, das aus den Zylindern 250, 260 und 270 sowie den stirnseitigen Deckelelementen 230 und 240 aufgebaut ist. Die Blutpumpe ist aus Förder- 10 - 60 und Antriebsmodul 70-150 zusammengesetzt.

Das Fördermodul wird zur Montage in den innersten Zylinder 250 des Oxygenators geschoben. Das Fördermodul wird durch die Schulterausformungen an der Öffnung des Deckels 230 und die Mutter 170 fixiert.

Das Antriebsmodul weist eine Schnellverschlussvorrichtung auf, die vorliegend als Bajonettverschluss, bestehend aus einer Steckerhalterung 110, die den Stecker 120 fixiert, einem Ringelement 130, einer Feder 140 und den Rückhohlstiften 150, ausgebildet ist. Beim Einschub der Einheit in das Oxygenationssystem rastet der Verschluss in die dafür vorgesehenen Nuten 400 im Zylinderelement 410, das mit dem Deckel 240 verbunden ist, ein. Durch die Feder wird das Antriebsmodul gegen das Rotormodul gepresst und dadurch axial fixiert. Auf Knopfdruck und bei gleichzeitiger Drehung rastet der Bajonettverschluss wieder aus. Durch die Feder springt der Druckknopf 100, bestehend aus den Elementen 110-150, aus dem Oxygenatorgehäuse, so dass die Antriebseinheit dem Oxygenator auf einfache und schnelle Weise entnommen werden kann.

Das Schaufelrad 10 wird über den Motor 90 mit Hilfe einer Magnetkupplung 40, 70 angetrieben. Als axiale Lagerung des Schaufelrades 10 dient die Stützlagerung 20, welches die Anziehungskräfte aus der Magnetkupplung 40, 70 aufnimmt. Die radiale Stabilisierung des Schaufelrades 10 wird berührungslos über die strömungsmechanische Lagerung 30 bewirkt, welche die Kippkräfte aus der Magnetkupplung 40, 70 aufnimmt.

Das Antriebsmodul besteht aus einem Motor 95 mit Magnetkupplung 70, achtpoligem Kabel und Stecker 120. Der Motor mit Magnetkupplung ist vollständig vom Motorgehäuse 90 mit aufgeschraubtem Motordeckel 80 umschlossen. Das Kabel mit Stecker wird aus dem Gehäuse herausgeführt. Dieser ist in einer mehrteiligen Vorrichtung 110-150 durch zwei Schrauben 370 so fixiert, dass eine direkte Konnektierung mit dem Gegenstecker der Stromversorgung von außen möglich ist. Das mittlere Bauteil der dreiteiligen Steckerhalterung ist ein Ringelement 130 mit zwei Bohrungen, in die zwei Passstifte für die Renkverbindung eingesetzt sind. Das größte Bauteil der Steckerhalterung 150 weist auf der dem Stecker abgewandten Seite drei Haken auf. Diese greifen in Schlitze im Motorgehäuse, halten so das Antriebsmodul zusammen und lassen trotzdem eine axiale Verschiebung gegenüber dem Motorgehäuse 90 zu. Die Feder 140 im Innern des Antriebsmoduls sorgt im eingebauten Zustand für eine spielfreie axiale Positionierung des Motorgehäuses 90 im Zylinder 250. Beim Lösen der Renkverbindung drückt sie den Stecker inklusive Halterung aus dem inneren Zylinder 250 heraus, während die restlichen Bauteile des Antriebsmoduls zunächst unverändert von der Anziehungskraft der Magnetkupplung in der Oxygenationseinheit gehalten werden. Die das System bedienende Person kann nun an der Steckerhalterung anfassen und das Antriebsmodul aus dem Oxygenator entfernen. Der Motor ist zweifach gegen Rotation in Folge des Motormoments gesichert. Unmittelbar wird das Moment vom Motorgehäuse aufgenommen, in das der Motor über den Motordeckel eingeklemmt ist. Das Motorgehäuse ist wiederum über die Renkverbindung gegen Verdrehen gesichert, das Drehmoment wird von dem Elementenpaar Nut/Stift aufgenommen.

Der Deckel ist zweiteilig aufgebaut und steckerseitig angeordnet. Eine Renknut 400 ist in einem separaten Zylinder 410 untergebracht, was die Fertigung erheblich vereinfacht. Der Zylinder ist mit dem Deckel 230 über ein Gewinde verbunden.

Die Strömungsführung erfolgt mittels strömungsmechanischer Lager 30. Die vom Schaufelrad 10 geförderte Strömung wird vorwiegend in den Strömungskanal zwischen dem ersten Zylinder 250 und dem zweiten 260 weitergeleitet. Ein Teil dieser Strömung wird nach Verlassen des Schaufelrades 10 in den Ringkanal zwischen dem Schaufelrad 10 und dem umgebenden Gehäuse abgezweigt. Diese retrograd gerichtete Strömung bewirkt eine radiale Stabilisierung des Schaufelrades 10.

Das Fördermodul besteht aus einem Förderelement 10, einer Lagerung 20, 30, Magneten 40, einer Kalotte 50, einer Bodenplatte 60, einem Einlass 160 und Ringelement 65 dargestellt. Diese Einheit kann wie das Antriebsmodul in das Membranmodul eingeschoben werden und bei Bedarf wieder entnommen werden.

In den dargestellten Ausführungsformen ist der Deckel mit anderen Bestandteilen verschraubt. Es ist grundsätzlich möglich, Bestandteile der Vorrichtung miteinander zu verschrauben. Dies hat den Vorteil einer einfacheren Demontierbarkeit.

Es ist jedoch gleichfalls möglich, Bestandteile der Vorrichtung auf beliebige andere Weise miteinander zu verbinden. Hierbei kommen sowohl lösbare Verbindungen, beispielsweise Klemmverbindungen oder Verbindungen durch Formschluss als auch nicht lösbare Verbindungen, z. B. durch Herstellung von Spritzgussteilen, durch Verkleben oder durch Verschweißen in Betracht.

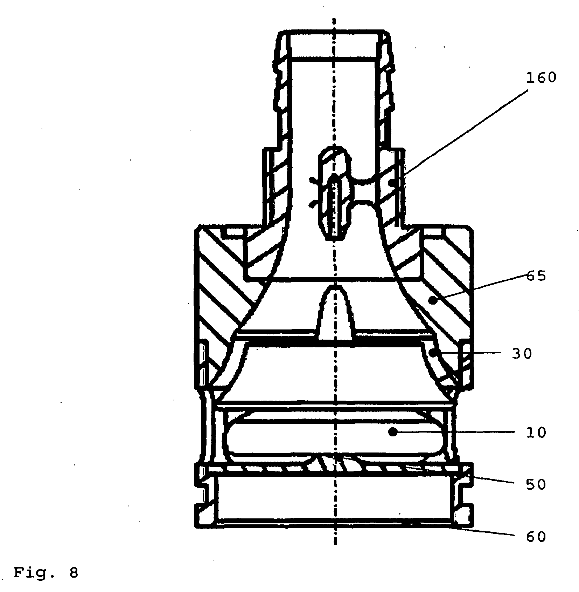

In Abbildung 8 ist ein Fördermodul bestehend aus Förderelement 10, Lagerung 20, 30, Magneten 40, Kalotte 50, Bodenplatte 60, Einlass 160 und Ringelement 65 dargestellt. Diese Einheit kann wie das Antriebsmodul in das Membranmodul eingeschoben werden und bei Bedarf wieder entnommen werden.

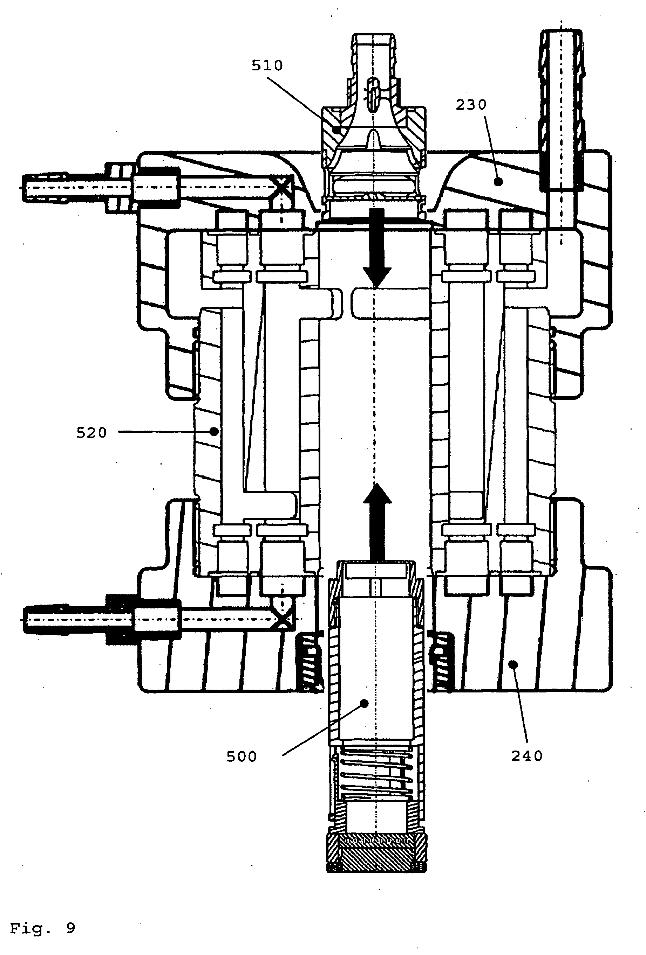

Die Pfeile in Abbildung 9 zeigen, in welche bevorzugte Richtung Antriebsmodul 500 und Fördereinheit 510 in den Oxygenator bestehend aus Membranmodul 520 und den beiden Deckeln 230, 240 eingeschoben werden.

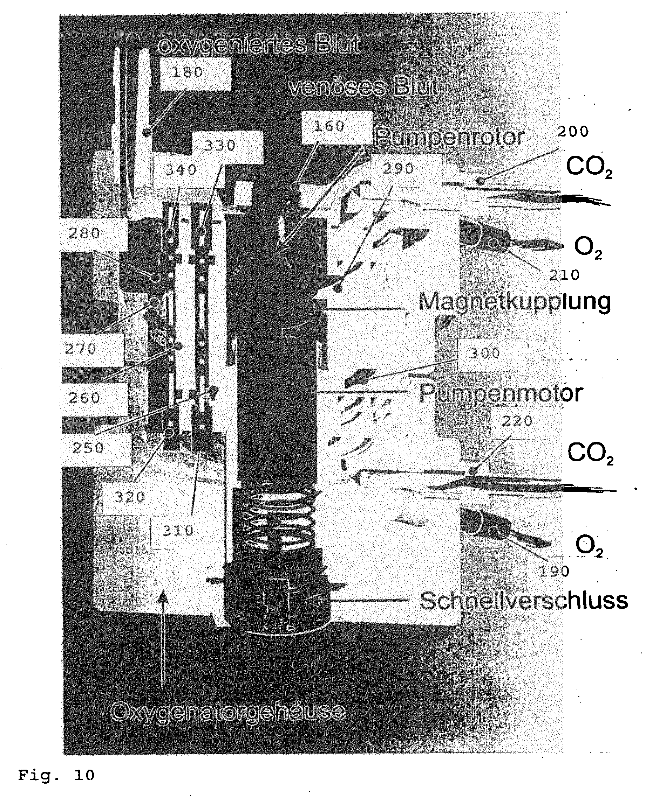

Abbildung 10 erläutert einen bevorzugten Verlauf von Blut - und Gasströmung. Im Einlass 160 tritt venöses Blut in das Oxygenationssystem - angesaugt durch die Blutpumpe - ein. Am Auslass 180 verlässt der mit Sauerstoff angereicherte Blutstrom das System. Sauerstoff oder Umgebungsluft strömt an den Anschlüssen 190 und 210 in das System und der mit Kohlendioxyd angereicherte Gasstrom verlässt das System an den Öffnungen 200 und 220.

Der Blutstrom und der Gasstrom werden zweimal zueinander im Gegenstrom geführt. Das Blut strömt durch den Einlass 160 in den Oxygenator. Dort strömt es - wie durch die Pfeile angedeutet - zunächst durch die Öffnung 290 im ersten Zylinder 250 in die erste Kammer 310, die an den Enden durch eine geeignete Verklebung verschlossen ist, hinein und dort an den semipermeablen Hohlfasermembranen 330 vorbei. Anschließend strömt es, nachdem es die Aussparungen 300 im zweiten Zylinder 260 passiert hat, in der zweiten Kammer 320, die auch an den Enden verklebt ist, in die entgegengesetzte Richtung. Durch die Passagen 280 im dritten Zylinder 270 strömt das Blut in den Blutdeckel und verlässt das System durch den Blutauslass 180. Die doppelte Gegenstromoxygenierung wird dadurch ermöglicht, dass Sauerstoff zunächst durch den Gaseinlass 190 in das System eintritt und anschließend durch diejenigen Membranen 330 in Kammer 310 strömt, die zwischen erstem Zylinder 250 und zweitem Zylinder 260 angeordnet sind. Dieser Gasstrom verlässt das Oxygenierungssystem durch den Auslass 200. Ein zweiter Gasstrom 210 wird zeitgleich durch die Membranen 340 in Kammer 320, zwischen zweitem Zylinder 260 und drittem Zylinder 270, geleitet und strömt durch den zweiten Gasauslass 220 wieder hinaus.

Eine besonders bevorzugte Ausführung der Vorrichtung weist technische Merkmale auf, die auf einem extrem kompakten und modularen Aufbau basieren und eine Lungen- (und/oder Herz)unterstützung mit ausreichender Sauerstoffversorgung und Kohlendioxydentfernung ermöglichen. Das erfindungsgemäße Oxygenationssystem wurde hinsichtlich guter Handhabbarkeit und Wiederverwertbarkeit entwickelt. Eine Blutpumpe, bestehend aus Fördermodul und Antriebsmodul, wird vollständig in das System hinein geschoben und durch einen Schnellverschluss fixiert und arretiert. Die Motoreinheit überträgt das Drehmoment auf den Rotor berührungslos durch eine Magnetkupplung. Der Rotor der Pumpe ist strömungsmechanisch und somit besonders blutschonend und für den Langzeiteinsatz geeignet, gelagert. Zudem ermöglicht diese Art der Lagerung den modularen Aufbau des Systems und somit eine leichte Montage und Wiederverwertbarkeit der Blutpumpeneinheit. Durch die Motorabwärme wird das Blut temperiert, so dass auf einen Wärmetauscher verzichtet werden kann. An den speziellen, koaxialen Anschluss kann eine Doppellumenkanüle zur minimalinvasiven Anwendung konnektiert werden.

Eine besonders bevorzugte Ausführung weist Mittel auf, die einen modularen Aufbau des Systems ermöglichen. Insbesondere ist zur zeitsparenden Montage der Rotorbereich so aufgebaut, dass sich der Rotor in einem Gehäuse befindet, das zur Montage leicht in das Oxygenationsmodul eingeschoben werden kann.

Die Abbildung 9 veranschaulicht eine besonders bevorzugte Durchführungsform des Vorgangs. Denkbar sind Ausführungen mit anderen Rotorformen oder Lagerungen, die dann - ohne das restliche System zu verändern - alternativ - je nach Anwendung - verwendet werden können.

Ebenso wie der Rotorkäfig ist auch das Antriebsaggregat auswechselbar. Insbesondere ist das Antriebsaggregat während des Betriebes rasch auswechselbar. Dies wird durch einen Schnellverschlussmechanismus ermöglicht. Besonders in kritischen Situationen, in denen schnell gehandelt werden muss, muss gewährleistet sein, dass ein defekter Antrieb gegen einen neuen unverzüglich ausgetauscht werden kann. Die Modulbauweise ermöglicht hier auch die Verwendung unterschiedlicher Antriebsaggregate. So kann z.B. in einer besonderen Ausführungsform der Antrieb eine Turbine sein, die zum Antrieb den Gasstrom aus der Sauerstoffflasche nutzt und nicht auf das Stromnetz oder ein Akkupaket angewiesen ist.

Im Gegensatz zur unteren Kugellagerung (mechanische Lagerung) liegt im Einlassbereich der Blutpumpe eine berührungslose Strömungslagerung des Rotors vor, welche das Laufrad in radialer Richtung stabilisiert. Die Vorteile der berührungslosen Radiallagerung sind neben der Verschleißminimierung insbesondere die geringere Gefahr der Thrombozytenaggregation und der Blutschädigung. Weiterhin werden der Fertigungsaufwand und damit auch die Produktionskosten entsprechend gesenkt, da der gesamte Aufbau des Pump- und Oxygenationssystems durch eine derartige Lagerung wesentlich vereinfacht wird.

Basierend auf der berührungslosen Radiallagerung ist ein schneller und unkomplizierter Einbau der Pumpe im Oxygenator möglich, da die Einbautoleranzen der Radiallagerung sehr groß gewählt werden können. Weiterhin wird die Anzahl der Pumpenbauteile verringert, welches gleichermaßen zu einer Reduzierung des Fertigungsaufwandes beiträgt.

Die Blutpumpe ist in das Gehäuse des Oxygenators so integriert, dass der Auslass der Pumpe direkt in den Einlass der Gasaustauscheinrichtung mündet. Durch diesen kompakten Aufbau werden Schlauchverbindungen zwischen Antriebssystem und Oxygenator vermieden. Dadurch wird das Füllvolumen des Systems reduziert.

Förder- und Antriebsmodul inklusive Magnetkupplung der modular aufgebauten integrierten Rotationsblutpumpe können durch die dafür vorgesehene Öffnung des Bodendeckels leicht in den Oxygenator hineingeschoben werden. Durch einen Schnellverschlussmechanismus wird die Antriebseinheit sicher arretiert und fixiert. Der Schnellverschluss kann in einer besonderen Ausführung ein Bajonettverschluss sein. Nach Gebrauch oder in kritischen Situationen (z. B. Ausfall der Pumpe) kann der Sicherheitsverschluss schnell entriegelt und die Antriebseinheit rasch gewechselt werden. Die Wiederverwertbarkeit der langlebigen Blutpumpeneinheit ist ressourcenschonend und ökologisch sehr vorteilhaft.

In einer bevorzugten Ausführungsform kann auf einen Wärmetauscher verzichtet werden, da die Motorwärme der integrierten Blutpumpe den Wärmeverlust des Blutstroms über die Oxygenatoroberfläche sowohl für kleine als auch für große Blutvolumenströme automatisch und ohne den Einsatz einer Steuerungseinheit so gut kompensiert, dass die physiologische Körpertemperatur im Blut aufrecht erhalten wird. Dadurch werden Füllvolumen und Fremdkontaktoberfläche des Oxygenators weiter verringert. Das Risiko für Patienten zu verbluten sowie für systemische, inflammatorische Reaktionen und Infektionen wird somit herabgesetzt.

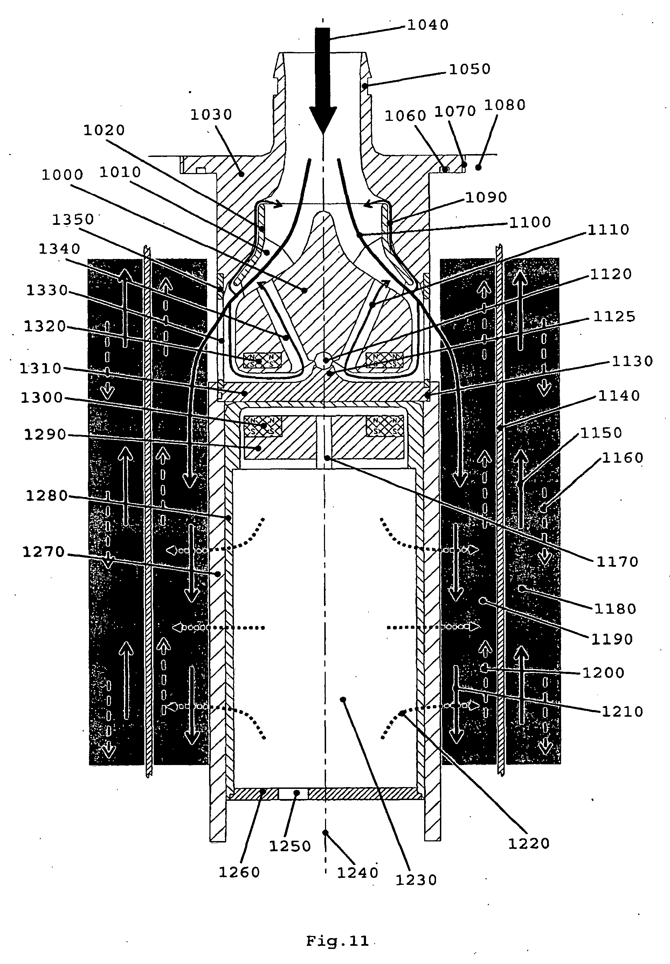

Figur 11 zeigt einen Oxygenator mit integrierter Blutpumpe 1000, 1010, 1020, 1030, 1050, 1070, 1310, 1350 und integrierter Antriebseinheit 1230, wobei die Übertragung der Drehmomente vom Antrieb 1230 auf das Laufrad berührungslos mit Hilfe einer permanentmagnetischen Axialkupplung 1300, 1320 erfolgt.

Die Blutströmung 1040 wird über den Pumpeneinlass 1050 in den Pumpe eingeleitet und durchströmt dann als Hauptströmung 1100 den beschaufelten Bereich 1010 des Laufrades 1000. Aufgrund des Druckaufbaus in der Pumpe resultieren neben der Hauptströmung 1100 weitere Sekundärströmungen 1090, 1110, die für den blutverträglichen Betrieb der Pumpe von zentraler Bedeutung sind. Der höhere Druck am Laufradaustritt hat zur Folge, dass ein Teil der Hauptströmung 1100 als Spülströmung 1110 am Laufradaustritt abgezweigt wird und den axialen Spaltraum zwischen der Rückseite des Laufrades 1000 und dem gegenüberliegenden Pumpendeckel durchströmt wird. Die Ausrichtung dieser Spülströmung 1110 ist aufgrund des Druckgefälles radial nach innen gerichtet und wird weiterhin über die Spülkanäle 1340, welche im Laufradkörper 1000 eingearbeitet sind, wieder auf die Laufradvorderseite weitergeleitet.

Auf diese Weise wird sowohl die für Thromenablagerungen kritische Laufradrückseite effektiv durchströmt und somit frei von Strömungsstagnationen gehalten, als auch die in diesem Bereich platzierte Pivot-Lagerung 1120, 1125 des Laufrades effizient umspült und somit gekühlt. Hieraus resultiert schließlich ein blutschonende Strömungsführung auf der Laufradrückseite.

Ein weiterer Teil der Hauptströmung 1100 wird ebenfalls infolge der Druckverteilung am Laufrad als Leckageströmung 1090 am Laufradaustritt abgezweigt und durchströmt von dort den radialen Spaltraum zwischen der Deckscheibe 1020, welche fest mit den Laufradschaufeln und/oder mit anderen Bestandteilen des Laufrades verbunden ist sowie ggf. mit dem gegenüberliegenden Pumpengehäuse 1030.

In einer besonders bevorzugten Ausführungsform wird diese Leckageströmung 1090 effektiv zur radialen Stabilisierung des Laufrades 1000 verwendet, wobei die Stabilsierung auf den vorherrschenden Fluidkräften im Spaltraum beruht. Die Funktionsweise dieses Radiallagers basiert vorwiegend auf einem "Lomakin-Effekt".

Bei konzentrischer Lage des Laufrades 1000 im Pumpengehäuse 1030 liegt im Lagerspalt 1090 ein über den Umfang konstanter statischer Druck vor. Wird das Laufrad jedoch in seitliche Richtungen ausgelenkt, so verengt sich der Lagerspalt auf der ausgelenkten Seite und vergrößert sich entsprechend auf der diametral gegenüberliegen Seite des Spaltes. Da der Druck im engeren Spaltbereich gegenüber dem Druck im diametral gegenüberliegenden Bereich aufgrund des unterschiedlichen Strömungswiderstandes ansteigt, resultiert auf die Deckscheibe 1020 und somit auch auf das Laufrad 1000 ein radiale Rückstellkraft, welche das Laufrad 1000 wieder in die konzentrische Lage im Pumpengehäuse 1030 versetzt. Es liegt somit ein radial wirkendes Lager vor, welches zusammen mit der Pivot-Lagerung - d.h. Kugelspurlagerung - 1120, 1125 eine komplette Rotorlagerung des Laufrades 1000 im Pumpengehäuse 1030 liefert, ohne dass es zu einer mechanische Berührung zwischen diesen beiden Komponenten kommt.
Der Antrieb des Laufrades basiert bei dieser Ausführung auf einer permanentmagnetisch wirkenden Axialkupplung 1300, 1320, welche in ihrer Funktionsweise einer Stirndrehkupplung gleichkommt. Aufgrund der axial anziehenden magnetischen Kräfte zwischen den Antriebsmagneten 1300 und den Abtriebsmagneten 1320 werden die vom Elektromotor 1230 zur Verfügung gestellten Drehmomente auf das Laufrad 1000 berührungslos übertragen. Die Antriebs- und Abtriebsmagnete bestehen jeweils aus einer geraden Anzahl von wechselseitig polarisierten Dauermagneten (z.B. NdFeB, SmCO, etc.). Bei Vorliegen eines Lastmomentes auf der Abtriebsseite werden die antriebseitigen Magnete 1300 gegenüber den Abtriebsmagneten 1320 soweit verdreht, bis das magnetische Luftspaltmoment gleich dem Lastmoment ist.

Die axialen Anziehungskräfte werden hierbei durch das Pivot-Lager 1120, 1125 aufgefangen und verhindern somit ein Aufschlagen des Laufrades 1000 am Pumpendeckel 1310.
Da das Laufrad 1000 jedoch in der Pivotlagerung 1120, 1125 gegen seitliche Verkippungen im Pumpengehäuse 1030 instabil gelagert ist bedarf es eines weiteren Radiallagers, welches über die oben beschriebene Fluidlagerung 1090 gemäß dem "Lomakin-Effekt" gewährleistet wird.

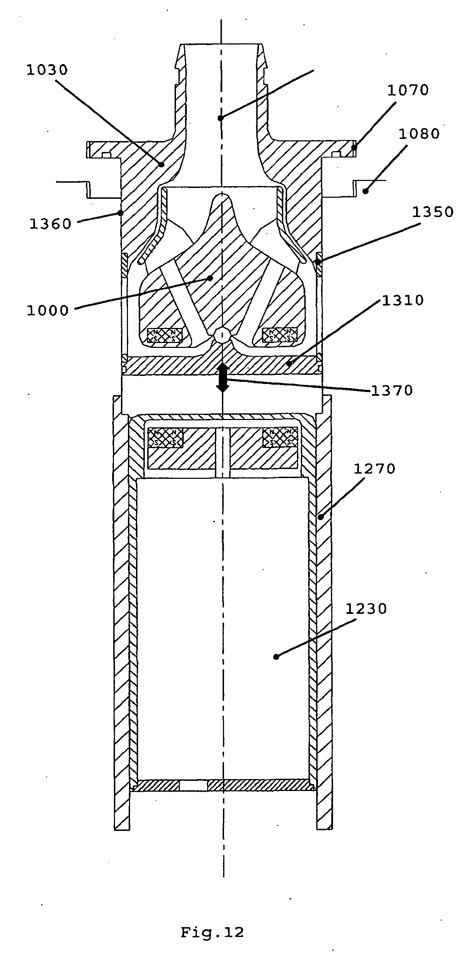

Figur 12 zeigt den Oxygenator aus Fig. 11, wobei der einfache Aus- und Einbau 1370 der Pumpeneinheit verdeutlicht ist. Der Ein - und Ausbau der modularen Pumpeneinheit wird beispielsweise wie in Fig. 12 wiedergegeben durch eine Schraubvorrichtung 1070 zwischen dem Pumpengehäuse 1030 und dem angrenzenden stationären Oxygenatorelement 1080 ermöglicht.

Diese Ein- und Ausbaufähigkeit der Pumpeneinheit, bietet den wesentlichen Vorteil, dass bei technischen Komplikationen (z.B. erhöhter Lagerverschleiß infolge hoher Betriebsleistungen der Blutpumpe) oder hämatologischen Komplikationen im Pumpenbereich (z.B. Thrombenablagerungen in der Blutpumpe) während des klinischen Einsatzes insbesondere bei langfristigen Anwendungen (z.B. ECMO) der Oxygenator durch einfaches Auswechseln der Pumpeneinheit weiterverwendet werden kann und der Patient somit nicht einer weiteren Oxygenationsbehandlung unterzogen werden muss, welches unter Umständen einen zusätzlichen chirurgischen Eingriff hinfällig macht und die ECMO-Behandlung beispielsweise dadurch in ihrer Gesamtheit patientenschonender durchgeführt werden kann.

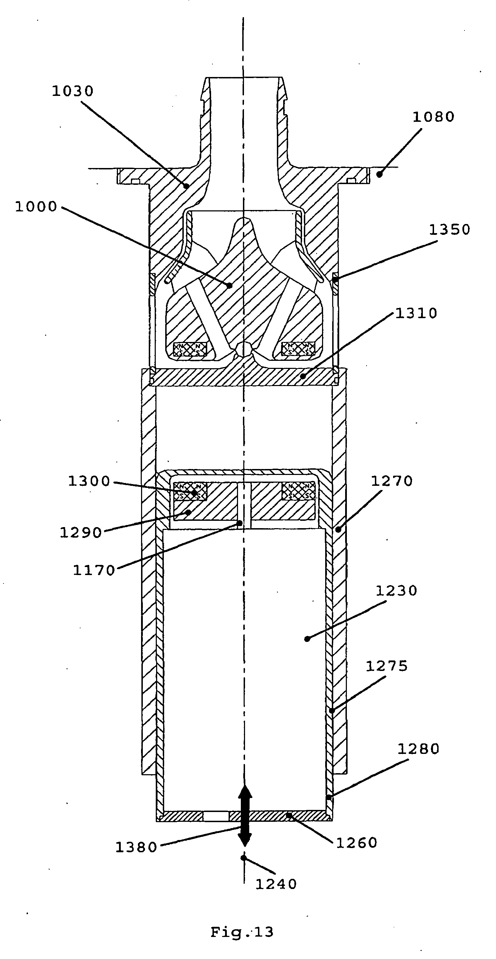

Figur 13 zeigt den Oxygenator aus Fig. 11, wobei der einfache Aus- und Einbau 1370 der Antriebseinheit verdeutlicht ist. Der Ein - und Ausbau der modularen Antriebseinheit wird durch einen geeigneten Schnellverschluss unterstützt. Diese Ein- und Ausbaufähigkeit der Antriebseinheit, bietet den wesentlichen Vorteil, dass das fertigungstechnisch aufwendige Antriebsmodul 1170,1230, 1280, 1260,1280, 1290, 1300 selbst bei einmaliger Verwendung des Oxygenationsmoduls stets für weitere Einsätze wieder verwendet werden kann, wodurch der Oxygenator insbesondere wirtschaftlicher eingesetzt werden kann.
Eine wesentliche Besonderheit bei diesem integrierten Antriebskonzept liegt ferner darin, dass die vom Motor in die Umgebung abgegebene Joulsche Verlustleistung effektiv zur Temperierung des Blutes im Oxygenationsmodul verwendet werden kann. Ein zusätzlicher Wärmetauscher, wie dieser gegenwärtig bei Blutoxygenationssystemen benötigt wird, kann bei dieser Ausführung des Oxygenators umgangen werden, so dass das Oxygenationssystem in seiner Gesamtheit kompakter wird und gleichermaßen einfacher zu bedienen ist.

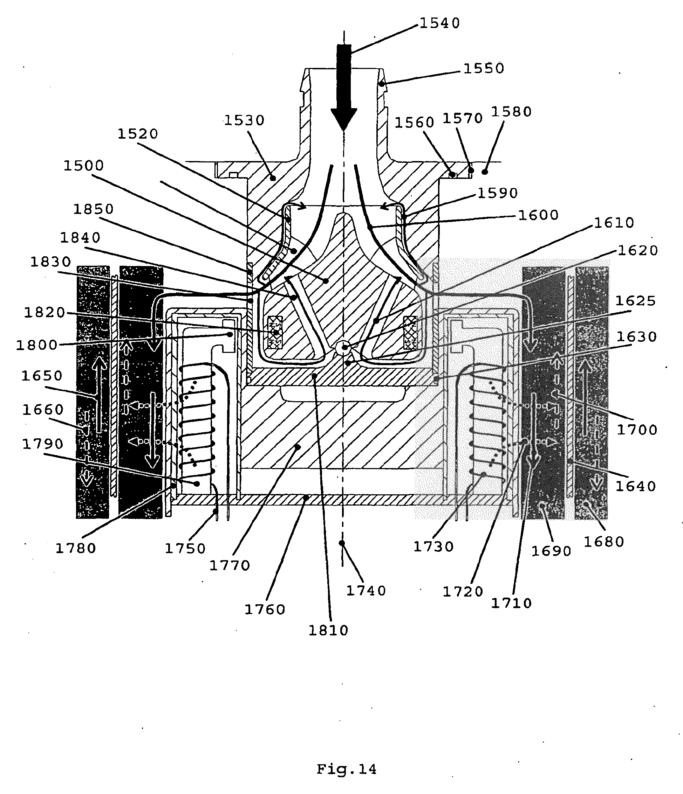

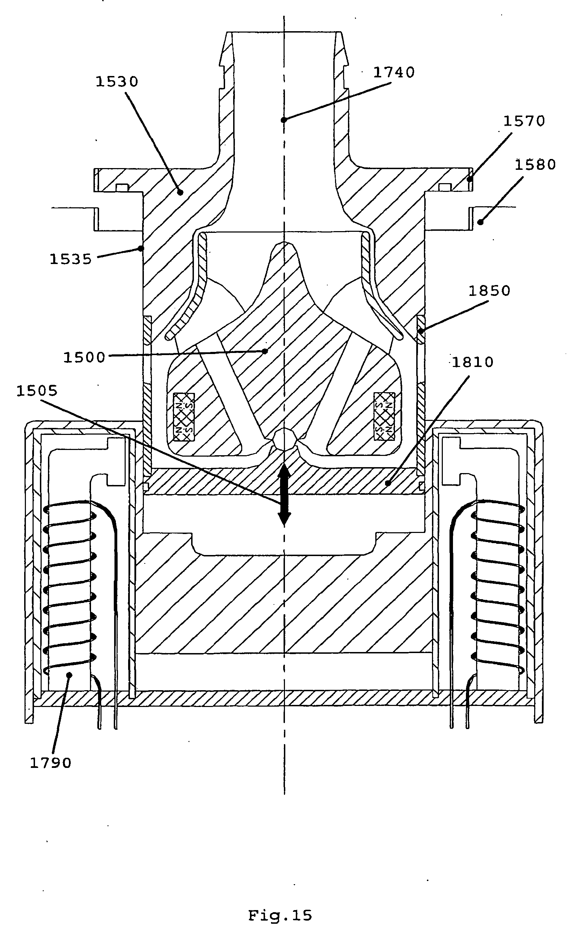

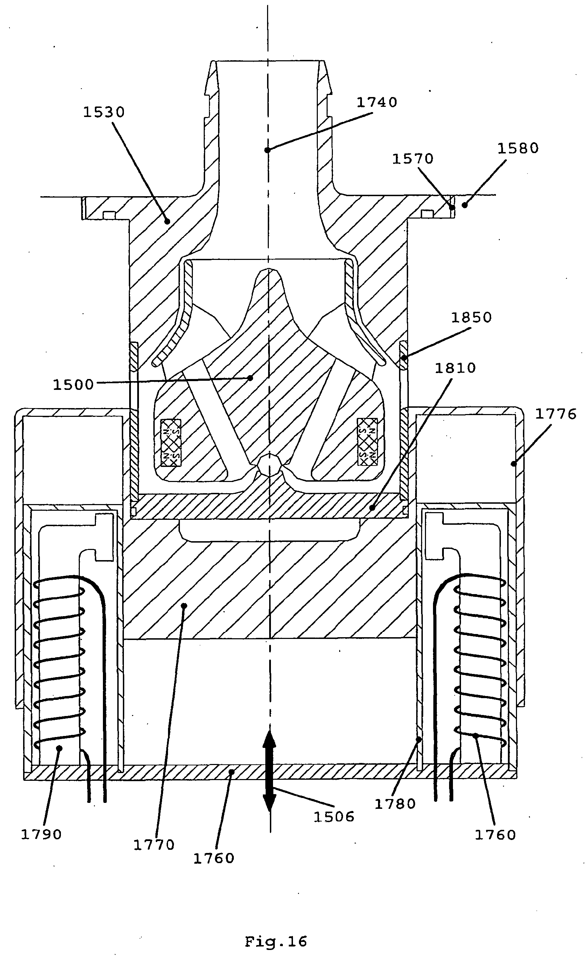

Figur 14 zeigt einen Oxygenator mit integrierter Blutpumpe 1500, 1510, 1520, 1530, 1550, 1570, 1810, 1850 und integrierter Antriebseinheit 1230, 1730, 1790, 1800, wobei die Übertragung der Drehmomente vom Antrieb 1800 auf das Laufrad 1500 berührungslos mit Hilfe einer elektromagnetischen Radialkupplung 1800, 1820 erfolgt. Der Aufbau und die Wirkungsweise dieser magnetischen Radialkupplung ist vergleichbar zu der in Fig. 11 dargestellten Axialmagnetkupplung, mit dem wesentlichen Unterschied, dass hier die Abtriebsmagneten 1820 nicht in axialer, sondern radialer Richtung magnetisiert sind. Auch hier stehen wieder eine gerade Anzahl von Dauermagnetsegmenten auf der Antriebs- und Abtriebsseite gegenüber und bewirken bei Anliegen eines Lastmomentes eine unter Verdrehung wirksam werdende berührungslose Drehmomentübertragung. Der wesentliche Vorteil eines Oxygenators gemäß der Ausführung als Fig. 14 besteht darin, dass die magnetische Radialkupplung gleichermaßen ein stabiles Axiallager zur Verfügung stellt, welches insbesondere die mechanischen Belastungen auf das Pivot-Lager reduziert. Die Nutzung der Joulschen Wärme für die Temperierung des Blutes und der Gase im Oxygenator bringt gleichermaßen die bereits bei Fig. 11 erwähnten Vorteile mit sich. Auch bei der Ausführung des Oxygenators gemäß Fig. 14 ist ein einfacher Ein- und Ausbau der Pumpeneinheit sowie der Antriebseinheit gegeben, wie dies beispielhaft in Fig. 15 und Fig. 16 dargestellt ist.

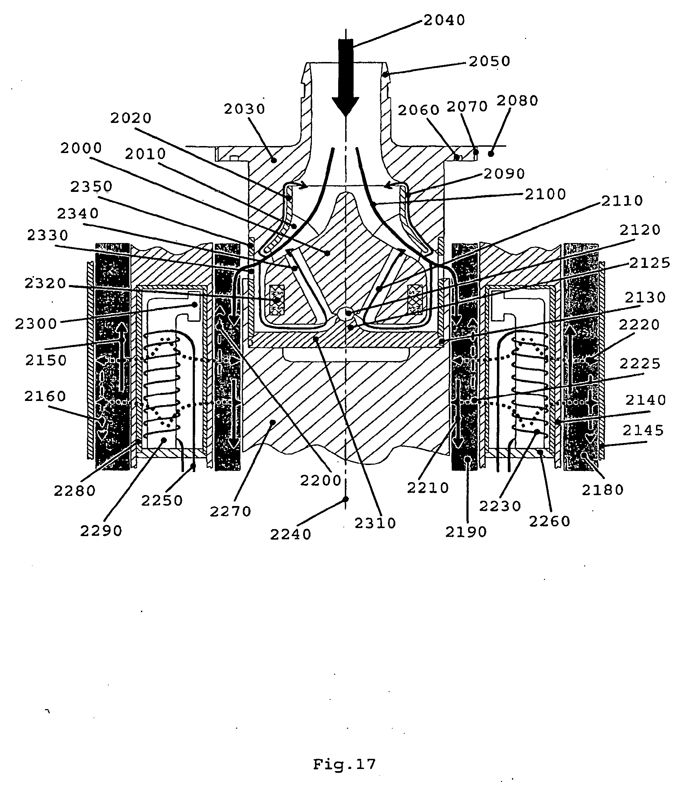

Figur 17 zeigt einen Oxygenator mit integrierter Blutpumpe 2000, 2010, 2020, 2030, 2050, 2070, 2310, 2350 und integrierter Antriebseinheit 2230, 2260, 2280, 2290, 2300, gemäss der Erfindung wobei die Übertragung der Drehmomente vom Antrieb 2300 auf das Laufrad 1500 berührungslos mit Hilfe einer elektromagnetischen Radialkupplung 1800, 1820 erfolgt. Der Aufbau und die Wirkungsweise dieser magnetischen Radialkupplung ist vergleichbar zu der in Fig. 14 dargestellten Radialmagnetkupplung, mit dem wesentlichen Unterschied, dass hier die Statoreinheit 2230, 2290, 2300 des Antriebes die in ihr erzeugte Joulsche Wärme nicht nur einseitig, wie bei Fig. 11 und Fig. 14, sondern zweiseitig an das Oxygenationsmodul 2180, 2190 abgibt. Dies wird ermöglicht durch die konzentrische Platzierung der Statoreinheit 2230, 2290, 2300 zwischen zwei Oxygenationsmodulen 2180, 2190, die im Falle des radial innen liegenden Oxygenationsmoduls 2190 die Joulsche Wärme über ihren zylindrischen Außenmantel und im Falle des radial außen liegenden Oxygenationsmoduls 2180 die Joulsche Wärme über ihren zylindrischen Innenmantel aufnimmt.

Hierdurch ist der wesentliche Vorteil gegeben, dass die Temperierung des Blutes, welches im extrakorporalen Kreislauf (ohne zusätzlichen Wärmetauscher) einer Abkühlung unterliegt, wesentlich effektiver umgesetzt wird.
Die weiteren Vorteile einer derartigen radialen Magnetkupplung insbesondere bezüglich ihrer Lager- und Stabilisierungsfunktion, können von den Ausführungen aus Fig. 14 übernommen werden.

Auch bei der Ausführung des Oxygenators gemäß Fig. 17 ist ein modularer Aufbau des Oxygenationssystems, bestehend aus Einwegteilen (Fasern, Membranen, etc.) sowie austauschbaren bzw. wiederverwendbaren Modulen (Pumpeneinheit, Antriebseinheit) gegeben. Die Vorteile eines modularen Aufbaus bei einem Oxygenator gemäß Fig. 17 können daher unmittelbar von denen aus Fig. 11 und Fig. 14 übernommen werden.

Besonders bevorzugte Ausführungsformen der Vorrichtung eignen sich beispielsweise für Patienten mit akutem Lungenversagen (ARDS). Hier ist die Extrakorporale Membran Oxygenation (ECMO) eine geeignete Hilfe. Bei dieser Therapie wird mittels Roller- oder Zentrifugalpumpen Blut durch einen Membranoxygenator gefördert. Durch semipermeable Membranen wird das Blut mit Sauerstoff angereichert und Kohlendioxyd abgereichert. Zur Steuerung der Bluttemperatur werden Wärmetauscher eingesetzt. Die Geräte werden ausschließlich stationär betrieben.

Durch die einfache Handhabbarkeit und die robuste und kompakte Ausführung kann die erfindungsgemäße Vorrichtung auch leicht transportiert werden und beispielsweise schon an einem Unfallort direkt appliziert werden. Dies erhöht die Überlebenschancen von Patienten mit sehr schweren Lungenschäden und gibt der Lunge die nötige Ruhe zum Heilen.

Durch das geringe Füllvolumen und die reduzierte Fremdkontaktoberfläche des hohen Füllvolumens reduziert die erfindungsgemäße Vorrichtung Risiken wie Infektionen, Schädigung der roten Blutkörperchen und Thrombozytenaggregation sowie das Risiko von Blutungen durch die notwendige Zugabe des Blutgerinnungshemmers Heparin. Durch den modularen Aufbau bevorzugter Ausführungsformen, die die wieder verwendbare Blutpumpeneinheit und die mit Blut in Berührung kommende Wegwerfeinheit - das Membranmodul - klar trennt, ist die Erfindung sowohl in Herstellung als auch in Betrieb als effektiv und effizient anzusehen.

### Bezugszeichenliste:

- 10: Schaufelrad
- 20: Stützlagerung
- 30: Strömungsmechanische Lagerung
- 40: Permanentmagnete
- 50: Kalotte
- 60: Bodenplatte des Fördermoduls
- 65: Ringelement
- 70: Magnetkupplung
- 80: Motordeckel
- 90: Motorgehäuse
- 95: Antriebsaggregat
- 100: Druckknopf
- 110: Steckerhalterung
- 120: Stecker
- 130: Ringelement mit Bohrungen
- 140: Federelement
- 150: Steckerhalterung
- 155: Rückholhaken
- 160: Bluteinlass
- 170: Schraube
- 180: Blutauslass
- 190: Gaseinlass
- 200: Gasauslass
- 210: Gaseinlass
- 220: Gasauslass
- 230: Deckel
- 240: Deckel (Boden)
- 250: Zylinder
- 260: Zylinder
- 270: Zylinder
- 280: Öffnungen
- 290: Öffnungen
- 300: Öffnungen
- 310: Kammer
- 320: Kammer
- 330: Fasermaterial
- 340: Fasermaterial
- 350: Verklebung
- 360: Verklebung
- 370: Schrauben
- 380: Passstifte
- 390: Schlitze
- 400: Renknut
- 410: Zylinderelement
- 500: Antriebsmodul
- 510: Fördereinheit
- 520: Membranmodul
- 1000: Laufrad
- 1010: Laufradschaufeln
- 1020: Deckscheibe
- 1030: Pumpengehäuse
- 1040: Zuströmung zur Blutpumpe bzw. zum Oxygenator
- 1050: Pumpeneinlass
- 1060: Dichtung (z.B. O-Ring-Dichtung)
- 1070: Gewinde
- 1080: stationäres Oxygenatorelement
- 1090: Leckageströmung bzw. Fluidlager
- 1100: Hauptströmung
- 1110: Spülströmung
- 1120: Lagerkugel (Pivot-Lager)
- 1125: Kalotte
- 1130: Dichtung (z.B. O-Ring-Dichtung)
- 1140: Trennelement zwischen den Oxygenatorfasern bzw. -membranen
- 1150: Blutströmung im außen liegenden Faserbündel
- 1160: Gasströmung im außen liegenden Faserbündel
- 1170: Motorwelle
- 1180: außen liegender Faserbündel
- 1190: innen liegender Faserbündel
- 1200: Gasströmung im innen liegenden Faserbündel
- 1210: Blutströmung im innen liegenden Faserbündel
- 1220: Wärmestrom vom Elektromotor zum Oxygenator
- 1230: Elektromotor
- 1240: Drehachse
- 1250: Ausleitung der Motorkabel
- 1260: Motordeckel
- 1270: Hohlzylinder als Trennung zwischen Motor und Oxygenator
- 1275: Gleitfläche zwischen Antriebseinheit und anliegendem stationärem Oxygenatorelement
- 1280: Motorgehäuse
- 1290: Polschuh der Magnetkupplung
- 1300: Antriebsmagnete
- 1310: Pumpendeckel
- 1320: Abtriebsmagnete
- 1330: Strömungskanal in der Auslassvorrichtung zur Blutpumpe
- 1340: Spülkanal
- 1350: Käfigförmige Auslassvorrichtung
- 1360: Gleitfläche zwischen Pumpeneinheit und anliegendem stationärem Oxygenatorelement
- 1370: Bewegungsrichtung beim Ein- und Ausbau der Pumpeneinheit
- 1380: Bewegungsrichtung beim Ein- und Ausbau der Antriebseinheit
- 1500: Laufrad
- 1505: Bewegungsrichtung beim Ein- und Ausbau der Pumpeneinheit
- 1506: Bewegungsrichtung beim Ein- und Ausbau der Antriebseinheit
- 1510: Laufradschaufeln
- 1520: Deckscheibe
- 1530: Pumpengehäuse
- 1535: Gleitfläche zwischen Pumpeneinheit und anliegendem stationärem Oxygenatorelement
- 1540: Zuströmung zur Blutpumpe bzw. zum Oxygenator
- 1550: Pumpeneinlass
- 1560: Dichtung (z.B. O-Ring-Dichtung)
- 1570: Gewinde
- 1580: stationäres Oxygenatorelement
- 1590: Leckageströmung bzw. Fluidlager
- 1600: Hauptströmung
- 1610: Spülströmung
- 1620: Lagerkugel (Pivot-Lager)
- 1625: Kalotte
- 1630: Dichtung (z.B. O-Ring-Dichtung)
- 1640: Trennelement zwischen den Oxygenatorfasern bzw. -membranen
- 1650: Blutströmung im außen liegenden Faserbündel
- 1660: Gasströmung im außen liegenden Faserbündel
- 1680: außen liegende Faserbündel
- 1690: innen liegende Faserbündel
- 1700: Gasströmung im innen liegenden Faserbündel
- 1710: Blutströmung im innen liegenden Faserbündel
- 1720: Wärmestrom vom Elektromotor zum Oxygenator
- 1730: Wicklungen (Statorspulen) des elektromagnetischen Antriebs
- 1740: Drehachse
- 1750: Ausleitung der Motorkabel
- 1760: Motordeckel
- 1770: stationäre Vorrichtung zum Trennen der Antriebseinheit vom Oxygenator
- 1776: Gleitfläche zwischen Antriebseinheit und anliegendem stationärem Oxygenatorelement
- 1780: Motorgehäuse
- 1790: Statormagnet des elektromagnetischen Antriebs
- 1800: Statormagnet gegenüberliegend zu Abtriebsmagneten
- 1810: Pumpendeckel
- 1820: Abtriebsmagnete
- 1830: Strömungskanal in der Auslassvorrichtung zur Blutpumpe
- 1840: Spülkanal
- 1850: Käfigförmige Auslassvorrichtung
- 2000: Laufrad
- 2005: Bewegungsrichtung beim Ein- und Ausbau der Pumpeneinheit
- 2006: Bewegungsrichtung beim Ein- und Ausbau der Antriebseinheit
- 2007: Vorrichtung innerhalb des Oxygenators, welches den axial beweglichen Antrieb als einheitliches Bauteil führt
- 2010: Laufradschaufeln
- 2020: Deckscheibe
- 2030: Pumpengehäuse
- 2035: Gleitfläche zwischen Pumpeneinheit und anliegendem stationärem Oxygenatorelement
- 2040: Zuströmung zur Blutpumpe bzw. zum Oxygenator
- 2050: Pumpeneinlass
- 2060: Dichtung (z.B. O-Ring-Dichtung)
- 2070: Gewinde
- 2080: stationäres Oxygenatorelement
- 2090: Leckageströmung bzw. Fluidlager
- 2100: Hauptströmung
- 2110: Spülströmung
- 2120: Lagerkugel (Pivot-Lager)
- 2125: Kalotte
- 2130: Dichtung (z.B. O-Ring-Dichtung)
- 2140: Trennelement zwischen den Oxygenatorfasern bzw. -membranen
- 2145: Abgrenzung der Oxygenatorfasern bzw. -membranen
- 2150: Blutströmung im außen liegenden Faserbündel
- 2160: Gasströmung im außen liegenden Faserbündel
- 2180: außen liegender Faserbündel
- 2190: innen liegender Faserbündel
- 2200: Gasströmung im innen liegenden Faserbündel
- 2210: Blutströmung im innen liegenden Faserbündel
- 2220: Wärmestrom vom Elektromotor zum außen liegenden Oxygenatorbündel
- 2225: Wärmestrom vom Elektromotor zum innen liegenden Oxygenatorbündel
- 2230: Wicklungen (Statorspulen) des elektromagnetischen Antriebs
- 2240: Drehachse
- 2250: Ausleitung der Motorkabel
- 2260: Motordeckel
- 2270: stationäre Vorrichtung zum Trennen der Antriebseinheit vom Oxygenator
- 2276: Gleitfläche zwischen Antriebseinheit und anliegendem stationärem Oxygenatorelement
- 2280: Motorgehäuse
- 2290: Statormagnet des elektromagnetischen Antriebs
- 2300: Statormagnet gegenüberliegend zu Abtriebsmagneten
- 2310: Pumpendeckel
- 2320: Abtriebsmagnete
- 2330: Strömungskanal in der Auslassvorrichtung zur Blutpumpe
- 2340: Spülkananl
- 2350: Käfigförmige Auslassvorrichtung

## Patentansprüche

1. Vorrichtung zur An- und/oder Abreicherung von Stoffen in einer Flüssigkeit, mit
- einem Membranmodul, welches im Wesentlichen aus konzentrischen Körpern besteht und ein Separationselement enthält, in dem der abzureichernde und/oder anzureichernde Stoff geführt ist, und wobei außerhalb des Separationselements die Flüssigkeit geführt ist;
- einem Antriebsmodul, welches eine Antriebseinheit (90,95) zum Antrieb eines die Flüssigkeit fördernden Förderelements (10) aufweist;
- einem Fördermodul zur Förderung der Flüssigkeit durch die Vorrichtung, mit
einem Förderelement (10), wobei das Antriebsmodul bei flüssigkeitsdichtem
Verschluss des Membranmoduls in dieses einschiebbar und herausnehmbar ist,
**dadurch gekennzeichnet,**
**dass** die Vorrichtung einen Oxygenator beinhaltet oder ein Oxygenator ist und dass der Oxygenator ein außen liegendes Faserbündel und ein innen liegendes Faserbündel aufweist, wobei zwischen dem außen liegenden und dem innen liegenden Faserbündel eine elektromagnetische Antriebseinheit integriert ist, welche eine radiale Magnetkupplung für ein zentrales innen liegendes Laufrad beinhaltet.

2. Vorrichtung nach Anspruch 1,
**dadurch gekennzeichnet, dass**
die Antriebseinheit integriert ist, welche sowohl das außen liegende als auch das innen liegende Faserbündel aufwärmt.

3. Vorrichtung nach einem der vorangegangenen Ansprüche,
**dadurch gekennzeichnet,**
**dass** das Separationselement hohle Fasern (330, 340) aus semipermeablem Material zur An- und/oder Abreicherung der Flüssigkeit aufweist, wobei in den Fasern der ab- und anzureichernde Stoff und außerhalb der Fasern die Flüssigkeit geführt ist.

4. Vorrichtung nach einem der vorangegangenen Ansprüche,
**dadurch gekennzeichnet,**
**dass** das Fördermodul zur Förderung der Flüssigkeit in axialer Verlängerung des Antriebsmoduls angeordnet ist.

5. Vorrichtung nach einem der vorangegangenen Ansprüche,
**dadurch gekennzeichnet,**
**dass** das Förderelement und ein das Förderelement umgebendes Gehäuse (Rotorgehäuse) voneinander trennbar angeordnet sind.

6. Vorrichtung nach einem der vorangegangenen Ansprüche,
**dadurch gekennzeichnet,**
**dass** durch einen flüssigkeitsdichten Abschluss des Förderelementes gegenüber einer Zuleitung der Flüssigkeit und/oder einer Ableitung der Flüssigkeit eine Auswechselbarkeit des Rotors ermöglicht wird.

7. Vorrichtung nach einem der vorangegangenen Ansprüche,
**dadurch gekennzeichnet,**
**dass** die Antriebseinheit so angeordnet ist, dass sie wenigstens abschnittsweise von der Flüssigkeit umgeben ist.

8. Vorrichtung nach Anspruch 7,
**dadurch gekennzeichnet,**
**dass** die Antriebseinheit so angeordnet ist, dass sie von der Flüssigkeit durch wenigstens eine Trennwand getrennt ist.

9. Vorrichtung nach einem der vorangegangenen Ansprüche,
**dadurch gekennzeichnet,**
**dass** die Antriebseinheit an wenigstens einem Ende einen Schnellverschluss (100, 110, 120, 130, 140, 150) aufweist.

10. Vorrichtung nach Anspruch 9,
**dadurch gekennzeichnet,**
**dass** eine Stirnseite der Antriebseinheit den Schnellverschluss (100, 110, 120, 130, 140, 150) aufweist.

11. Vorrichtung nach einem der Ansprüche 9 oder 10,
**dadurch gekennzeichnet,**
**dass** der Schnellverschluss (100, 110, 120, 130, 140, 150) ein Bajonettverschluss ist.

12. Vorrichtung nach einem der Ansprüche 9 oder 10,
**dadurch gekennzeichnet,**
**dass** der Schnellverschluss (100, 110, 120, 130, 140, 150) ein Schraubverschluss ist.

13. Vorrichtung nach einem der Ansprüche 9 oder 10,
**dadurch gekennzeichnet,**
**dass** der Schnellverschluss (100, 110, 120, 130, 140, 150) ein Klemmverschluss ist.

14. Vorrichtung nach einem der Ansprüche 9 oder 10,
**dadurch gekennzeichnet,**
**dass** der Schnellverschluss (100, 110, 120, 130, 140, 150) ein Magnetverschluss ist.

15. Vorrichtung nach einem der vorangegangenen Ansprüche,
**dadurch gekennzeichnet,**
**dass** zwischen der Antriebseinheit und einer Rotoreinheit ein Dämpfungselement angeordnet ist.

16. Vorrichtung nach einem der vorangegangenen Ansprüche,
**dadurch gekennzeichnet,**
**dass** das Membranmodul wenigstens zwei konzentrisch zueinander angeordnete Körper (250, 260, 270) aufweist, wobei das Separationselement (330, 340) zwischen einem ersten (250) Körper und einem zweiten Körper (260) angeordnet ist.

17. Vorrichtung nach Anspruch 16,
**dadurch gekennzeichnet,**
**dass** das Membranmodul wenigstens drei konzentrisch zueinander angeordnete Körper (250, 260, 270) aufweist, wobei das Separationselement (330, 340) zwischen dem ersten Körper (250) und dem zweiten Körper (260) angeordnet ist und wobei ein weiteres Separationselement (330, 340) zwischen dem zweiten Körper (260) und dem dritten Körper (270) angeordnet ist.

18. Vorrichtung nach einem der Ansprüche 16 oder 17,
**dadurch gekennzeichnet,**
**dass** die Räume zwischen den Körpern (250, 260, 270) an den Stirnseiten flüssigkeitsdicht verschlossen sind.

19. Vorrichtung nach einem der Ansprüche 16 bis 18,
**dadurch gekennzeichnet,**
**dass** das Fördermodul geometrisch so ausgestaltet ist, dass es im Innenraum des Innersten der drei Körper (250, 260, 270) platzierbar und in diesen einschiebbar und herausnehmbar ist.

20. Vorrichtung nach einem der Ansprüche 16 bis 19,
**dadurch gekennzeichnet,**
**dass** der innerste der drei Körper (250) des Membranmoduls an der dem Fördermodul gegenüberliegenden Seite einen Deckel (100) mit einem Schnellverschluss aufweist.

21. Vorrichtung nach einem der vorstehenden Ansprüche,
**dadurch gekennzeichnet,**
**dass** der radiale Außendurchmesser des Fördermoduls kleiner als der Radius des Innenraums des innersten Körpers (250) ist.

22. Vorrichtung nach Anspruch 21,
**dadurch gekennzeichnet,**
**dass** das Fördermodul bei der Montage der Vorrichtung in den Innenraum des innersten Körpers (250) einschiebbar ist.

23. Vorrichtung nach einem der vorstehenden Ansprüche,
**dadurch gekennzeichnet,**
**dass** auf eine der Stirnseiten eines der Zylinder ein Aufsatz aufsetzbar ist, welcher eine Zuführung (160) und eine Abführung zur Zu- und Abführung der Flüssigkeit aufweist, die koaxial zueinander angeordnet sind.

24. Vorrichtung nach einem der vorstehenden Ansprüche,
**dadurch gekennzeichnet,**
**dass** die zwischen dem ersten und zweiten Zylinder platzierten und zwischen dem zweiten und dritten Zylinder platzierten hohlen Fasern jeweils eine Stoffzu- und eine Stoffableitung aufweisen.

25. Vorrichtung nach Anspruch 24,
**dadurch gekennzeichnet,**
**dass** die Gaszuführung (190) der zwischen dem ersten und zweiten Körper platzierten hohlen Fasern an einer Stirnseite der Körper und die Gaszuführung (210) der zwischen dem zweiten und dritten Körper platzierten Fasern an der gegenüberliegenden Stirnseite der Körper platziert sind.

26. Vorrichtung zur An- und/oder Abreicherung von Stoffen in einer Flüssigkeit nach einem der vorangegangenen Ansprüche, mit einer Antriebseinheit (90, 95) zum Antrieb eines die Flüssigkeit fördernden Förderelements (10),
**dadurch gekennzeichnet,**
**dass** die Kraftübertragung von der Antriebseinheit (90, 95) auf das Förderelement (10) berührungslos ist.

27. Vorrichtung nach Anspruch 26,
**dadurch gekennzeichnet,**
**dass** die Kraftübertragung von der Antriebseinheit (90, 95) auf das Förderelement (10) mittels einer Magnetkupplung (40, 70) stattfindet.

28. Vorrichtung nach einem der vorstehenden Ansprüche,
**dadurch gekennzeichnet,**
**dass** die Vorrichtung zur Aufnahme der Antriebseinheit (90, 95) im Wesentlichen eine zylinderförmige Aufnahme (250) aufweist.

29. Vorrichtung nach einem der vorstehenden Ansprüche,
**dadurch gekennzeichnet,**
**dass** die Antriebseinheit (90, 95) im Betrieb Wärme abgibt.

30. Vorrichtung nach Anspruch 29,
**dadurch gekennzeichnet,**
**dass** die Antriebseinheit (90, 95) ein Motor ist.

31. Vorrichtung nach einem der vorstehenden Ansprüche
**dadurch gekennzeichnet,**
**dass** die Antriebseinheit (90, 95) in einem Wärme leitenden Kontakt mit der zylinderförmigen Aufnahme (250) steht.

32. Vorrichtung nach Anspruch 31,
**dadurch gekennzeichnet,**
**dass** die Flüssigkeit an der Außenseite der zylinderförmigen Aufnahme (250) entlang geführt wird.

33. Vorrichtung nach einem der vorstehenden Ansprüche,
**dadurch gekennzeichnet,**
**dass** das Förderelement (10) in radialer Richtung strömungsmechanisch gelagert ist.

34. Vorrichtung nach Anspruch 33,
**dadurch gekennzeichnet,**
**dass** die strömungsmechanische Lagerung in einem Raum zwischen Förderelement (10) und umgebendem Gehäuse durch einen dem Hauptförderstrom entgegensetzten Sekundärstrom bewirkt wird.

35. Vorrichtung nach einem der Ansprüche 33 bis 34,
**dadurch gekennzeichnet,**
**dass** das Förderelement (10) auf der dem Antriebsmodul zugewandten Seite in einem körperhaften Axiallager aufgelagert ist.

36. Vorrichtung nach einem der vorhergehenden Ansprüche,
**dadurch gekennzeichnet,**
**dass** sie wenigstens zwei Antriebsmodule beinhaltet.

37. Vorrichtung nach Anspruch 36,
**dadurch gekennzeichnet,**
**dass** zwei Antriebsmodule in Reihenschaltung angeordnet sind.

38. Vorrichtung nach Anspruch 36 oder 37,
**dadurch gekennzeichnet,**
**dass** zwei Antriebsmodule in Parallelschaltung angeordnet sind.

## Claims

1. A device for enriching and/or depleting substances in a liquid, having:
- a membrane module which essentially consists of concentric members and which contains a separating element in which the substance to be enriched and/or depleted is guided, and wherein the liquid is guided outside the separating element;
- a drive module which comprises a drive unit (90, 95) for driving a conveying element (10) which conveys the liquid,
- a conveying module for conveying the liquid through the device, having
- a conveying element (10), wherein the drive module can be inserted into and removed from the membrane module when the membrane module is closed in a liquid-tight manner,
***characterized in that***
the device contains an oxygenator or is an oxygenator and **in that** the oxygenator has an outer fibre bundle and an inner fibre bundle; wherein an electromagnetic drive unit which contains a radial magnetic coupling for a central internally located impeller is integrated between the outer fibre bundle and the inner fibre bundle.

2. The device as claimed in claim 1, ***characterized in that*** the drive unit is integrated in a manner such as to warm up both the outer fibre bundle and the inner fibre bundle.

3. The device as claimed in one of the preceding claims, ***characterized in that*** the separating element comprises hollow fibres (330, 340) produced from semi-permeable material for enriching and/or depleting the liquid, wherein the substance to be depleted and/or enriched is guided in the fibres and the liquid is guided outside the fibres.

4. The device as claimed in one of the preceding claims, ***characterized in that*** the conveying module for conveying the liquid is disposed in an axial extension of the drive module.

5. The device as claimed in one of the preceding claims, ***characterized in that*** the conveying element and a housing (rotor housing) which surrounds the conveying element are capable of being separated from each other.

6. The device as claimed in one of the preceding claims. ***characterized in that*** the rotor is replaceable because the conveying element is sealed in a liquid-tight manner with respect to a feed line for the liquid and/or with respect to a discharge line for the liquid.

7. The device as claimed in one of the preceding claims, ***characterized in that*** the drive unit is disposed in a manner such as to be at least partially surrounded by the liquid.

8. The device as claimed in claim 7, ***characterized in that*** the drive unit is configured in a manner such as to be separated from the liquid by at least one partition.

9. The device as claimed in one of the preceding claims, ***characterized in that*** the drive unit comprises a quick-release closure (100, 110, 120, 130, 140, 150) on at least one end thereof.

10. The device as claimed in claim 9, ***characterized in that*** the quick-release closure (100, 110, 120, 130, 140, 150) is positioned at one end of the drive unit.

11. The device as claimed in claim 9 or 10, ***characterized in that*** the quick-release closure (100, 110, 120, 130, 140, 150) is a bayonet coupling.

12. The device as claimed in claim 9 or 10, ***characterized in that*** the quick-release closure (100, 110, 120, 130, 140, 150) is a screw closure.

13. The device as claimed in claim 9 or 10, ***characterized in that*** the quick-release closure (100, 110, 120, 130, 140, 150) is a clamp-type closure.

14. The device as claimed in claim 9 or 10, ***characterized in that*** the quick-release closure (100, 110, 120, 130, 140, 150) is a magnetic closure.

15. The device as claimed in one of the preceding claims, ***characterized in that*** a shock-absorbing element is disposed between the drive unit and a rotor unit.

16. The device as claimed in one of the preceding claims, ***characterized in that*** the membrane module comprises at least two members (250, 260, 270) disposed concentrically with respect to each other, wherein the separating element (330, 340) is disposed between a first member (250) and a second member (260).

17. The device as claimed in claim 16, ***characterized in that*** the membrane module comprises at least three members (250, 260, 270) disposed concentrically with respect to each other, wherein the separating element (330, 340) is disposed between the first member (250) and the second member (260), and wherein a further separating element (330, 340) is disposed between the second member (260) and the third member (270).

18. The device as claimed in claim 16 or 17, ***characterized in that*** the spaces between the members (250, 260, 270) are sealed at the ends in a liquid-tight manner.

19. The device as claimed in one of claims 16 to 18, ***characterized in that*** the conveying module is geometrically configured in a manner such that it can be positioned in the interior of the innermost of the three members (250, 260, 270) and can be inserted into it and removed therefrom.

20. The device as claimed in one of claims 16 to 19, ***characterized in that*** the innermost (250) of the three members of the membrane module comprises a cover (100) with a quick-release closure on the side opposite to the conveying module.

21. The device as claimed in one of the preceding claims, ***characterized in that*** the radially external diameter of the conveying module is smaller than the radius of the interior of the innermost member (250).

22. The device as claimed in claim 21, ***characterized in that*** the conveying module can be inserted into the interior of the innermost member (250) during assembly of the device.

23. The device as claimed in one of the preceding claims, ***characterized in that*** a cap can be positioned on one of the ends of the cylinder, the cap having a feed line (160) and a discharge line disposed coaxially with respect to each other for feeding and discharging the liquid.

24. The device as claimed in one of the preceding claims, ***characterized in that*** the hollow fibres positioned between the first and second cylinder and between the second and third cylinder each comprise a substance feed line and a substance discharge line.

25. The device as claimed in claim 24, ***characterized in that*** the gas feed line (190) of the hollow fibres positioned between the first and second members is positioned at one end of the member and the gas feed line (210) for the fibres positioned between the second and third members is positioned at the opposite end of the member.

26. A device for enriching and/or depleting substances in a liquid as claimed in one of the preceding claims, having a drive unit (90, 95) for driving a conveying element (10) which conveys the liquid, ***characterized in that*** transmission of the force from the drive unit (90, 95) to the conveying element (10) is contact-free.

27. The device as claimed in claim 26, ***characterized in that*** the force is transmitted from the drive unit (90, 95) to the conveying element (10) by means of a magnetic coupling (40, 70).

28. The device as claimed in one of the preceding claims, ***characterized in that*** the device has an essentially cylindrical compartment (250) to accommodate the drive unit (90, 95).

29. The device as claimed in one of the preceding claims, ***characterized in that*** the drive unit (90, 95) produces heat during operation.

30. The device as claimed in claim 29, ***characterized in that*** the drive unit (90, 95) is a motor.

31. The device as claimed in one of the preceding claims, ***characterized in that*** the drive unit (90, 95) is in heat-conducting contact with the cylindrical compartment (250).

32. The device as claimed in claim 31, ***characterized in that*** the liquid is guided along the outside of the cylindrical compartment (250).

33. The device as claimed in one of the preceding claims, ***characterized in that*** the conveying element (10) is fluid-mechanically mounted in the radial direction.

34. The device as claimed in claim 33, ***characterized in that*** the fluid-mechanical mounting is provided by means of a secondary flow which runs counter to the main conveying flow in a space between the conveying element (10) and the surrounding housing.

35. The device as claimed in claim 33 to 34. ***characterized in that*** the conveying element (10) is mounted in a solid axial bearing in the side facing the drive module.

36. The device as claimed in one of the preceding claims, ***characterized in that*** the device comprises at least two drive modules.

37. The device as claimed in claim 36, ***characterized in that*** two drive modules are connected in series.

38. The device as claimed in claim 36 or 37, ***characterized in that*** the two drive modules are connected in parallel.

## Revendications

1. Dispositif permettant d'enrichir et/ou d'appauvrir un liquide en matières, comportant
- un module à membrane qui est essentiellement constitué de corps concentriques et qui contient un élément de séparation dans lequel la matière à appauvrir et/ou enrichir est conduite alors que ledit liquide est conduit à l'extérieur de l'élément de séparation ;
- un module d'entraînement comportant une unité d'entraînement (90, 95) servant à entraîner un élément de refoulement (10) destiné à refouler ledit liquide,
- un module de refoulement servant à refouler ledit liquide à travers ledit dispositif et comportant un élément de refoulement (10),
- le module d'entraînement pouvant être introduit par coulissement dans le module à membrane et en être retiré alors que ce dernier est fermé de manière à faire l'étanchéité aux liquides,
***caractérisé en ce que***
ledit dispositif comprend un organe d'oxygénation ou constitue un organe d'oxygénation et que l'organe d'oxygénation comporte un faisceau de fibres disposé à l'extérieur et un faisceau de fibres disposé à l'intérieur, une unité d'entraînement électromagnétique étant intégrée entre le faisceau de fibres disposé à l'extérieur et celui disposé à l'intérieur tout en comprenant un organe d'accouplement magnétique radial destiné à une roue centrale disposée à l'intérieur.

2. Dispositif selon la revendication 1, ***caractérisé en ce que*** l'unité d'entraînement est intégrée tout en chauffant tant le faisceau de fibres disposé à l'extérieur que celui disposé à l'intérieur.

3. Dispositif selon l'une des revendications précédentes, ***caractérisé en ce que*** l'élément de séparation comporte des fibres creuses (330, 340) en un matériau semi-perméable pour enrichir et/ou appauvrir ledit liquide, la matière à appauvrir et enrichir étant conduite à l'intérieur desdites fibres et ledit liquide étant conduit à l'extérieur des fibres.

4. Dispositif selon l'une des revendications précédentes, ***caractérisé en ce que*** le module de refoulement, destiné à refouler ledit liquide, est disposé dans le prolongement axial du module d'entraînement.

5. Dispositif selon l'une des revendications précédentes, ***caractérisé en ce que*** l'élément de refoulement et un boîtier entourant l'élément de refoulement (boîtier de rotor) sont disposés séparément l'un de l'autre.

6. Dispositif selon l'une des revendications précédentes, ***caractérisé en ce qu***'en réalisant un fermeture étanche aux liquides de l'élément de refoulement vis-à-vis d'une ligne d'alimentation en ledit liquide et/ou d'une ligne d'évacuation dudit liquide, le rotor peut être remplacé.

7. Dispositif selon l'une des revendications précédentes, ***caractérisé en ce que*** l'unité d'entraînement est disposée de manière à être entourée, au moins sur certaines parties, dudit liquide.

8. Dispositif selon la revendication 7, ***caractérisé en ce que*** l'unité d'entraînement est disposée de manière à être séparée dudit liquide par au moins une cloison.

9. Dispositif selon l'une des revendications précédentes, ***caractérisé en ce que*** l'unité d'entraînement comporte, au moins sur l'une de ses extrémités, une fermeture rapide (100, 110, 120, 130, 140, 150).

10. Dispositif selon la revendication 9, ***caractérisé en ce que*** l'unité d'entraînement comporte, sur une de ces faces frontales, une fermeture rapide (100, 110, 120, 130, 140, 150).

11. Dispositif selon l'une des revendications 9 ou 10, ***caractérisé en ce que*** la fermeture rapide (100, 110, 120, 130, 140, 150) est une fermeture à baïonnette.

12. Dispositif selon l'une des revendications 9 ou 10, ***caractérisé en ce que*** la fermeture rapide (100, 110, 120, 130, 140, 150) est une fermeture à vis.

13. Dispositif selon l'une des revendications 9 ou 10, ***caractérisé en ce que*** la fermeture rapide (100, 110, 120, 130, 140, 150) est une fermeture fonctionnant par serrage.

14. Dispositif selon l'une des revendications 9 ou 10, ***caractérisé en ce que*** la fermeture rapide (100, 110, 120, 130, 140, 150) est une fermeture magnétique.

15. Dispositif selon l'une des revendications précédentes, ***caractérisé en ce qu*'**un élément amortisseur est disposé entre l'unité d'entraînement et une unité rotor.

16. Dispositif selon l'une des revendications précédentes, ***caractérisé en ce que*** le module à membrane comporte au moins deux corps (250, 260, 270) en disposition concentrique les uns par rapport aux autres, l'élément de séparation (330, 340) étant disposé entre un premier (250) corps et un deuxième corps (260).

17. Dispositif selon la revendication 16, ***caractérisé en ce que*** le module à membrane comporte au moins trois corps (250, 260, 270) en disposition concentrique les uns par rapport aux autres, l'élément de séparation (330, 340) étant disposé entre le premier corps (250) et le deuxième corps (260) et un élément de séparation supplémentaire (330, 340) étant disposé entre le deuxième corps (260) et le troisième corps (270).

18. Dispositif selon l'une des revendications 16 ou 17, ***caractérisé en ce que*** les espaces entre les corps (250, 260, 270) sont fermés, sur leurs faces frontales, de manière à faire l'étanchéité aux liquides.

19. Dispositif selon l'une des revendications 16 à 18, ***caractérisé en ce que*** le module de refoulement est réalisé de manière à avoir une géométrie permettant de le placer dans l'espace intérieur de celui des trois corps (250, 260, 270) qui est situé le plus à l'intérieur et de l'introduire dans celui-ci par coulissement et de l'en retirer.

20. Dispositif selon l'une des revendications 16 à 19, ***caractérisé en ce que*** celui des trois corps (250) du module à membrane qui est situé le plus à l'intérieur présente, sur son côté opposé au module de refoulement, un couvercle (100) pourvu d'une fermeture rapide.

21. Dispositif selon l'une des revendications précédentes, ***caractérisé en ce que*** le diamètre extérieur radial du module de refoulement est inférieur au rayon de l'espace intérieur du corps (250) situé le plus à l'intérieur.

22. Dispositif selon la revendication 21, ***caractérisé en ce que,*** lors du montage dudit dispositif, le module de refoulement peut être introduit par coulissement dans l'espace intérieur du corps (250) situé le plus à l'intérieur.

23. Dispositif selon l'une des revendications précédentes, ***caractérisé en ce que,*** sur l'une des faces frontales d'un des cylindres, on peut mettre en place en embout comportant une ligne d'alimentation (160) et une ligne d'évacuation lesquelles permettent d'introduire et de faire sortir ledit liquide et lesquelles sont en disposition coaxiale l'une par rapport à l'autre.

24. Dispositif selon l'une des revendications précédentes, ***caractérisé en ce que*** les fibres creuses, placées entre le premier et le deuxième cylindre et placées entre le deuxième et le troisième cylindre, comportent chacune une ligne d'alimentation en matière et une ligne d'évacuation de matière.

25. Dispositif selon la revendication 24, ***caractérisé en ce que*** la ligne d'alimentation en gaz (190) de la fibre creuse placée entre le premier et le deuxième corps est placée à l'une des faces frontales des corps, et la ligne d'alimentation en gaz (210) de la fibre creuse placée entre le deuxième et le troisième corps est placée à la face frontale opposée des corps.

26. Dispositif d'enrichissement et/ou d'appauvrissement de matières contenues dans un liquide selon l'une des revendications précédentes, comportant une unité d'entraînement (90, 95) destinée à entraîner un élément de refoulement (10) refoulant ledit liquide, ***caractérisé en ce que*** la transmission de force depuis l'unité d'entraînement (90, 95) vers l'élément de refoulement (10) est réalisée sans contact.

27. Dispositif selon la revendication 26, ***caractérisé en ce que*** la transmission de force depuis l'unité d'entraînement (90, 95) vers l'élément de refoulement (10) est réalisée au moyen d'un organe d'accouplement (40, 70) magnétique.

28. Dispositif selon l'une des revendications précédentes, ***caractérisé en ce que,*** pour accueillir l'unité d'entraînement (90, 95), ledit dispositif présente un logement (250) de forme essentiellement cylindrique.

29. Dispositif selon l'une des revendications précédentes, ***caractérisé en ce que,*** lors de son fonctionnement, l'unité d'entraînement (90, 95) dégage de la chaleur.

30. Dispositif selon la revendication 29, ***caractérisé en ce que*** l'unité d'entraînement (90, 95) est un moteur.

31. Dispositif selon l'une des revendications précédentes, ***caractérisé en ce que*** l'unité d'entraînement (90, 95) en contact avec le logement (250) de forme cylindrique de manière à conduire la chaleur.

32. Dispositif selon la revendication **31, *caractérisé en ce que*** ledit liquide est conduit le long de la face extérieure du logement (250) de forme cylindrique.

33. Dispositif selon l'une des revendications précédentes, ***caractérisé en ce que*** dans le sens radial, l'élément de refoulement (10) est disposé conformément à la mécanique des fluides.

34. Dispositif selon la revendication 33, ***caractérisé en ce que,*** dans un espace entre l'élément de refoulement (10) et le boîtier l'entourant, la disposition conforme à la mécanique des fluides est le résultat d'un flux secondaire de refoulement allant à l'encontre du flux principal de refoulement.

35. Dispositif selon l'une des revendications 33 à 34, ***caractérisé en ce que*** l'élément de refoulement (10) prend appui, sur le côté faisant face au module d'entraînement, dans un palier à butée axiale constituant un corps.

36. Dispositif selon l'une des revendications précédentes, ***caractérisé en ce qu*'**il comprend au moins deux modules d'entraînement.

37. Dispositif selon la revendication 36, ***caractérisé en ce que*** les deux modules d'entraînement sont montés en série.

38. Dispositif selon les revendications 36 ou 37, ***caractérisé en ce que*** les deux modules d'entraînement sont montés en parallèle.
